# EUROPEAN PATENT SPECIFICATION

(11) **EP 1 446 489 B1**
(45) Date of publication and mention of the grant of the patent: **18.01.2012**
(21) Application number: 02783361.5
(22) Date of filing: 15.10.2002
(51) Int. Cl.: C12N 15/85

(54) **INTACT MINICELLS AS VECTORS FOR DNA TRANSFER AND GENE THERAPY IN VITRO AND IN VIVO**
INTAKTE MINIZELLEN ALS VEKTOREN FÜR DNA-TRANSFER UND GENTHERAPIE IN VITRO UND IN VIVO
MINI-CELLULES ENTIERES UTILISEES EN TANT QUE VECTEURS POUR LE TRANSFERT D'ADN ET LA THERAPIE GENIQUE IN VITRO ET IN VIVO

(30) Priority: 15.10.2001 US 328801 P
(43) Date of publication of application: 18.08.2004
(60) Divisional application: 10176209.4
(73) Proprietor: EnGeneIC Molecular Delivery Pty Ltd., Sydney NSW 2066 (AU)
(72) Inventor: BRAHMBHATT, Himanshu, Sydney, NSW 2176 (AU); MACDIARMID, Jennifer, Glebe, Sydney, NSW 2037 (AU)
(74) Representative: Inspicos A/S
(86) International application number: PCT/IB2002/004632
(87) International publication number: WO 2003/033519

(56) References cited:
- WO-A-03/072014
- WO-A-2004/113507
- US-A- 4 497 796
- KHACHATOURIANS G G ET AL: "A NEW METHOD FOR THE PREPARATION OF MINICELLS FOR PHYSIOLOGICAL STUDIES" PREPARATIVE BIOCHEMISTRY, NEW YORK, NY, US, vol. 3, no. 3, 1973, pages 291-298, XP009066765
- TANNY G B ET AL: "IMPROVED FILTRATION TECHNIQUE FOR CONCENTRATING AND HARVESTING BACTERIA" APPLIED AND ENVIRONMENTAL MICROBIOLOGY, WASHINGTON,DC, US, vol. 40, no. 2, August 1980 (1980-08), pages 269-273, XP009037575 ISSN: 0099-2240
- CARDEW P ET AL: "Scale-up of crossflow filters: Reducing the risks" FILTRATION AND SEPARATION, CROYDON, GB, vol. 32, no. 6, June 1995 (1995-06), pages 493-498, XP004137392 ISSN: 0015-1882
- SUZUKI ET AL.: 'Production in escherichia coli of biologically active secretin, a gastrointestinal hormone' PROC. NATL. ACAD. SCI. USA vol. 79, 1982, pages 2475 - 2479, XP002400466
- CHRISTEN ET AL.: 'Rapid isolation of escherichia coli minicells by glass-fiber filtration: study of plasmid-coded polypeptides' GENE vol. 23, no. 2, August 1983, pages 195 - 198, XP003000864
- FORBES: 'Crossflow microfiltration' AUSTRALIAN JOURNAL OF BIOTECHNOLOGY vol. 1, no. 1, 1987, pages 30 - 33, XP001247235
- FOX ET AL.: 'Fate of the DNA in plasmid-containing escherichia coli minicells ingested by human neutrophils' BLOOD vol. 69, no. 5, 1987, pages 1394 - 1400, XP008070709

## Description

### BACKGROUND OF THE INVENTION

The present invention relates to the delivery, by means of intact bacterial minicells, of oligonucleotides and polynucleotides to host cells, particularly but not exclusively in the context of gene therapy. The invention also relates to a pharmaceutically compatible method for purifying intact bacterial minicells.

A U.S. patent to Salser et al., No. 4,497,796, illuminates various early approaches, available *circa* 1980, for transforming mammalian cells. In particular, Salser *et al.* disclose transfer of a gene encoding dihydrofolate reductase, which confers methotrexate resistance, into mouse L1210 cells or bone marrow cells, by the technique of DNA co-precipitation with calcium phosphate.

Salser *et al.* also mention a "number of [other] ways...for insertion of genetic materials into cells," including, in addition to "viral vectors," certain cell-fusion techniques: "cell-cell fusion involving the fusion of cells of a limited number of chromosomes enveloped in nuclear membranes;...minicell fusion;...fusion with bacterial protoplasts containing plasmid DNA; and fusion with erythrocyte ghosts packaged with DNA" (column 5, lines 18 - 30; citations omitted). Common to these techniques is the use of a DNA-containing cellular structure, delimited by a single membrane, that is brought into contact with a target mammalian cell in the presence of a membrane-fusion promoting agent, such as polyethylene glycol (PEG). The mammalian cell and the cellular structure fuse, forming a hybrid cell, and the DNA contained in the latter is released into the cytoplasm of the former and transported to the nucleus of the hybrid, which then may express the DNA.

The Salser disclosure mentions "erythrocyte ghosts," erythrocyte remnants that are devoid of cytoplasmic contents but that retain original morphology, and "bacterial protoplasts," or bacterial cells from which the outer membrane (cell wall) has been stripped, typically by the action of a lytic enzyme or an antibiotic that inhibits peptidoglycan synthesis. Salser *et al.* also allude to the use of a third type of simplified cellular structure, a minicell protoplast.

A minicell is an anucleate form of an *E. coli* or other bacterial cell, engendered by a disturbance in the coordination, during binary fission, of cell division with DNA segregation. Prokaryotic chromosomal replication is linked to normal binary fission, which involves mid-cell septum formation. In *E. coli,* for example, mutation of *min* genes, such as *min*CD, can remove the inhibition of septum formation at the cell poles during cell division, resulting in production of a normal daughter cell and an anulceate minicell (de Boer *et al.,* 1992; Raskin & de Boer, 1999; Hu & Lutkenhaus, 1999; Harry, 2001).

In addition to *min* operon mutations, anucleate minicells also are generated following a range of other genetic rearrangements or mutations that affect septum formation, for example in the *div*IVB1 in *B. subtilis* (Reeve and Cornett, 1975; Levin *et al.,* 1992). Minicells also can be formed following a perturbation in the levels of gene expression of proteins involved in cell division/chromosome segregation. For example, overexpression of *min*E leads to polar division and production of minicells. Similarly, chromosome-less minicells may result from defects in chromosome segregation for example the *smc* mutation in *Bacillus subtilis* (Britton *et al.,* 1998), *spo*OJ deletion in *B. subtilis* (Ireton *et al.,* 1994), *muk*B mutation in *E. coli* (Hiraga *et al.,* 1989), and *parC* mutation in *E. coli* (Stewart and D'Ari, 1992). Gene products may be supplied *in trans.* When over-expressed from a high-copy number plasmid, for example, CafA may enhance the rate of cell division and/or inhibit chromosome partitioning after replication (Okada *et al.,* 1994), resulting in formation of chained cells and anucleate minicells (Wachi *et al.,* 1989; Okada *et al.,* 1993).

Minicells are distinct from other small vesicles that are generated and released spontaneously in certain situations and, in contrast to minicells, are not due to specific genetic rearrangements or episomal gene expression. Exemplary of such other vesicles are bacterial blebs, which are small membrane vesicles (Dorward *et al.,* 1989). Blebs have been observed in several bacterial species from *Agrobacterium, Bacillus, Bordetella, Escherichia, Neisseria, Pseudomonas, Salmonella* and *Shigella,* for example. Bacterial blebs can be produced, for instance, through manipulation of the growth environment (Katsui *et al.,* 1982) and through the use of exogenous membrane-destabilizing agents (Matsuzaki *et al.,* 1997).

Other sub-bacterial components exist, such as bacterial ghosts (Lubitz *et al.,* 1999), which are empty bacterial envelopes formed, in a variety of Gram-negative bacteria when the phiX174 lysis gene E is expressed. Bacterial ghosts are formed from a transmembrane tunnel structure, through a bacterial cell envelope. Due to high osmotic pressure inside the cell, cytoplasmic content is expelled into the surrounding media, leading to an empty bacterial cell envelope.

Because plasmid replication within prokaryotic cells is independent of chromosomal replication, plasmids can segregate into both normal daughter cells and minicells during the aberrant cell division described above. Thus, minicells derived from recombinant *min E. coli* carry significant numbers of plasmid copies, with all of the bacterial cellular components except for chromosomes, and have been used as such in studying plasmid-encoded gene expression *in vitro.* See Brahmbhatt (1987), Harlow *et al.* (1995), and Kihara *et al.* (1996). Brahmbhatt (1987) demonstrated, for example, that *E. coli* minicells can carry recombinant plasmids with DNA inserts as large as 20 kb, absent any chromosomal DNA, and can express nine or more recombinant proteins simultaneously.

Such non-reproductive but metabolically active, intact minicells have been employed for analyzing proteins encoded by plasmid-borne genes. In the context of the "minicell fusion" mentioned by Salser *et al.,* however, minicells are stripped of their outer membranes to yield a structure that, like a bacterial protoplast, can undergo fusion with a target cell when both are incubated together with PEG or another agent which promotes cell fusion. Also like bacterial protoplasts, such minicell protoplasts must be maintained under isotonic conditions, in order to prevent osmotic lysis, and they are highly vulnerable to enzymatic attack. Thus, they are unsuitable for gene therapy. Further, with the advent of other, more convenient transformation methodology, the minicell technology referred to by Salser *et al.,* fell into disuse.

More recently, the advance of gene therapy has highlighted a variety of methods for introducing exogenous genetic material into the genome of a recipient mammal. See reviews by Romano *et al.* (1998, 1999), Balicki and Beutler (2002), and Wadhwa *et al.* (2002). The clinical application of these techniques, such as the utilization of adenovirus or recombinant retrovirus vectors, has been delayed because of serious safety concerns. Illustrative of the problems presented by transformation methodology now are recombination with wild-type viruses, insertional and oncogenic potential, virus-induced immunosuppression, limited capacity of the viral vectors to carry large segments of therapeutic DNA, reversion to virulence of attenuated viruses, difficulties in recombinant virus manufacture and distribution, low stability, and adverse reactions, such as an inflammatory response, caused by existing immunity. An approach that obviated these problems would offer significant benefit in making gene therapy safer and more effective.

Live attenuated bacterial vectors also are being explored as gene delivery vectors for human gene therapy, including *Salmonella* (Darji *et al.,* 1997; Paglia *et al.,* 2000; Urashima *et al.,* 2000), *Shigella* (Sizemore *et al.,* 1995; Grillot-Courvalin *et al.,* 2002), *Listeria* (Dietrich *et al.,* 1998) and invasive *E. coli* (Grillot-Courvalin *et al.,* 1998). However, bacterial vectors have significant limitations because live bacteria, though attenuated, must be engineered to carry phagolysosome membrane lysis mechanisms, to enable sufficient recombinant DNA to escape to the mammalian cell cytosol and hence the nucleus. Such engineering is difficult and may be impossible for many intracellular bacterial pathogens. Moreover, mutations that attenuate bacterial pathogens are known for only a few bacterial species, for example mutations in the aromatic amino acid biosynthesis genes for *Salmonella, E. coli* and *Shigella.*

Because attenuating mutations are not known for many bacterial species, a bacterial gene delivery system cannot exploit the vast battery of bacterial intracellular pathogens. Bacterial vectors raise an additional concern regarding the presence of chromosomal DNA because parts of this DNA could be transferred to other microflora in the human or animal host receiving the gene therapy. Such promiscuous transfer of DNA between bacterial species is undesirable due to a potential for emergence of new virulent and/or drug resistant bacteria.

### SUMMARY OF THE INVENTION

To address these and other needs, the present invention provides, in accordance with one aspect, a composition comprising (i) recombinant, intact (i) recombinant, intact bacterial minicells and (ii) a pharmaceutically acceptable carrier therefor, wherein said intact bacterial minicells contain a plasmid comprising a DNA sequence encoding a therapeutic peptide or polypeptide expression product, for example interleukin 2 and wherein said composition is free of contaminants 0.2 µm or less in size. In a preferred embodiment, the composition contains fewer than one contaminating parent cell per 10⁷, 10⁸, or 10⁹ minicells.

According to another aspect, the present invention provides for a use of recombinant, intact bacterial minicells in the preparation of a medicament that comprises intact bacterial minicells containing a plasmid comprising a DNA sequence encoding a therapeutic peptide or polypeptide expression product, wherein said medicament is free of contaminants 0.2 µm or less in size, for use in a method of (i) treating a disease, allergy or infection or (ii) modifying a trait, by administration of said medicament to a cell, tissue, or organ, wherein said disease is selected from (1) cancer; (2) an acquired disease selected from AIDS, pneumonia and emphysema; and (3) a condition engendered by an inborn error of metabolism, the allergy or infection is treated by modification of an immune response, and said trait is fertility.

In related aspects the invention pertains to recombinant, intact bacterial minicells containing a plasmid comprising a DNA sequence encoding a therapeutic peptide or polypeptide expression product, wherein said bacterial minicells are free of contaminants 0.2 µm or less in size , for use as a pharmaceutical.

Further an aspect of the invention relates to a composition of the invention as described above for use in a method of (i) treating a disease, allergy or infection or (ii) modifying a trait, by administration of said composition to a cell, tissue, or organ, wherein said disease is selected from (1) cancer; (2) an acquired disease selected from AIDS, pneumonia and emphysema; and (3) a condition engendered by an inborn error of metabolism, the allergy or infection is treated by modification of an immune response, and said trait is fertility.

The invention also provides, pursuant to a further aspect, a genetic transformation method that comprises (i) providing recombinant, intact bacterial minicells that contain a plasmid comprising a DNA sequence encoding a therapeutic peptide or polypeptide expression product, wherein said bacterial minicells are free of contaminants 0.2 µm or less in size and (ii) bringing said preparation of intact bacterial minicells into contact *in vitro* with non-phagocytic endocytosis-competent mammalian cells, such that said bacterial minicells are engulfed by said mammalian cells, whereby said mammalian cells produce said expression product. The aforementioned plasmid also may contain a second nucleic acid segment that functions as a regulatory element, such as a promoter, a terminator, an enhancer or a signal sequence, and that is operably linked to the first nucleic acid segment. Further, the plasmid may contain a reporter element, such as a nucleic acid segment coding for green fluorescent protein.

In accordance with yet another aspect of the present invention, a purification method is provided that comprises passing a sample containing intact bacterial minicells (i) over a series of cross-flow filters and then (ii) through a dead-end filter, whereby said intact bacterial minicells are separated from contaminants in said sample to obtain a purified minicell preparation. The method optionally includes a treatment of the purified minicell preparation with an antibiotic. Also optional is a preliminary step of performing differential centrifugation on the minicell-containing sample. In a preferred embodiment, the series of cross-flow filters comprises (A) at least one or two filters that employ a pore size greater than or equal to about 0.45 µm and (B) at least one filter employing a pore size less than or equal to about 0.2 µm.

### BRIEF DESCRIPTION OF THE DRAWINGS

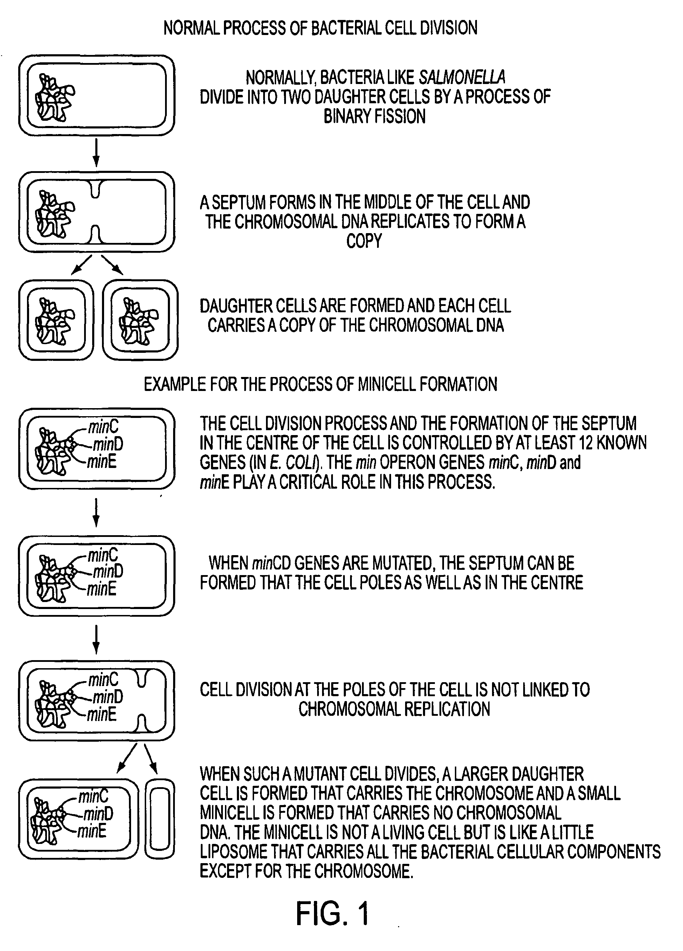

**Figure 1** shows a schematic illustration of normal cell division in a bacterium and how mutations in the *min* genes result in the formation of minicells.
**Figure 2** shows a schematic diagram of plasmid preparation useful for generating a minicell-producing bacterial strain. In particular, this figure shows a plasmid construction to generate a *S*. *typhimurium-derived* minicell producing strain. Line drawings of cloned insert DNAs and parts of circular plasmid vectors are shown. Plasmid names are shown in bold to the left of insert DNAs and inside circular vector maps. Where PCR primers are used in generating a clone, the primer numbers are shown adjacent to the dashed arrow.
**Figure 3** shows a schematic diagram of plasmid preparation useful for generating a minicell-producing bacterial strain. In particular, this figure shows a plasmid construction to generate a *Shigella flexneri* 2*a*-derived, minicell producing strain.
**Figure 4** shows a schematic diagram of plasmid preparation useful for generating a minicell-producing bacterial strain. In particular, this figure shows a plasmid construction to generate a *Listeria monocytogenes*-derived, minicell producing strain.
**Figure 5** shows TEM images of mouse macrophage cells transfected with *S*. *typhimurium*-derived minicells. For each panel, the time post-infection and the magnification are provided. The images show minicell-like electron dense particles (arrows) within macrophage vacuoles.

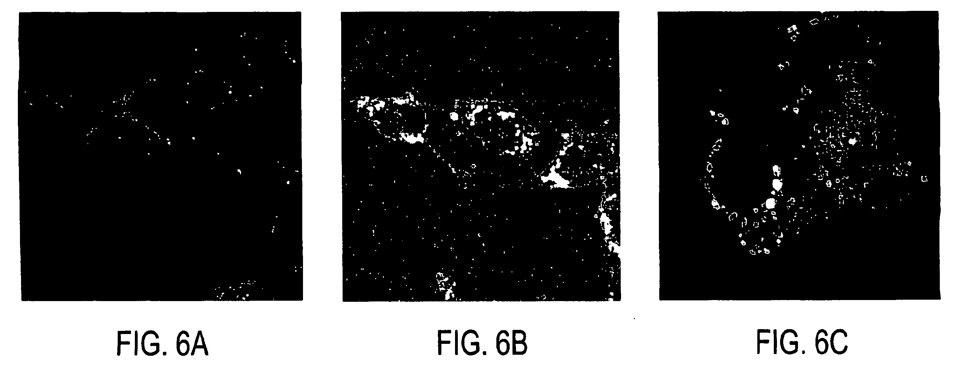

**Figure 6** shows minicell-mediated gene delivery to human breast cancer cells and heterologous gene expression. Panel A shows control breast cancer cell line SK-BR-3 96 hours post-transfection with non-recombinant minicells and labeled anti-HER-2 antibody, detected with Alexafluor-conjugated secondary antibody. Panel B shows GFP expression in SK-BR-3 cells, 96 hours post-transfection with recombinant minicells carrying plasmid pEGFP-C1 (eukaryotic expression of GFP). Panel C shows minicell/pEGFP-C1-transfected SK-BR-3 cells, labeled with anti-HER-2 antibody and detected with Alexafluor-conjugated secondary antibody. The images were visualized with confocal microscopy.
**Figure 7** shows mouse serum anti-GFP response 14 days after intraperitoneal administration of recombinant minicells and killed *S*. *typhimurium* parent cells carrying plasmid pEGFP-C1. In the legend and in the legends of Figures 8 - 10, 10*8 and 10*9 represent 10⁸ and 10⁹, respectively.
**Figure 8** shows mouse serum anti-LPS response 14 days after intraperitoneal administration of recombinant minicells, and killed *S*. *typhimurium* parent cells that carry plasmid pEGFP-C1.
**Figure 9** shows mouse serum anti-GFP response, 14 days after intraperitoneal administration of recombinant minicells carrying plasmid pEGFP-C1.
**Figure 10** shows mouse serum anti-LPS response, 14 days after intraperitoneal administration of recombinant minicells carrying plasmid pEGFP-C1.

### DETAILED DESCRIPTION OF PREFERRED EMBODIMENTS

The present inventors have determined that minicells with intact cell walls ("intact minicells") are effective vectors for delivering oligonucleotides and polynucleotides (collectively, "nucleic acid molecules") to host cells (preferably mammalian cells in humans or animals) *in vitro* and *in vivo.* Thus, the inventors have found that an intact minicell is engulfed by mammalian cells that likewise engulf the bacterial cell from which the minicell was obtained (the "parent bacterial cell"). Further, it has been discovered that, surprisingly, the contents of an intact, recombinant minicell, when engulfed by a host cell, are processed in such a way that at least some plasmid DNA from the minicell escapes degradation and is transported through the cytoplasm to the nucleus of the host cell, which then can express the plasmid DNA.

In accordance with the present invention, therefore, an intact minicell that carries a DNA for which heterologous expression is desired, either *in vitro* or *in vivo,* is brought into contact with a mammalian cell that engulfs the parent bacterial cell, in the manner of the intracellular bacterial pathogens. By the same token, that mammalian cell, denoted here as "engulfing-competent," engulfs the minicell and expresses the DNA in question.

A variety of mechanisms may be involved in the engulfing of intact minicells by a given type of host cell, and the present invention is not dependent on any particular mechanism in this regard. For example, phagocytosis is a well-documented process in which macrophages and other phagocyte cells, such as neutrophils, ingest particles by extending pseudopodia over the particle surface until the particle is totally enveloped. Although described as "non-specific" phagocytosis, the involvement of specific receptors in the process has been demonstrated. See Wright & Jong (1986); Speert *et al.* (1988).

Thus, one form of phagocytosis involves interaction between surface ligands and ligand-receptors located at the membranes of the pseudopodia (Shaw and Griffin, 1981). This attachment step, mediated by the specific receptors, is thought to be dependent on bacterial surface adhesins. With respect to less virulent bacteria, such as non-enterotoxigenic *E. coli,* phagocytosis also may occur in the absence of surface ligands for phagocyte receptors. See Pikaar *et al.* (1995), for instance. Thus, the present invention encompasses but is not limited to the use of intact minicells that either possess or lack surface adhesins, in keeping with the nature of their parent bacterial cells, and are engulfed by phagocytes (*i.e.,* "phagocytosis-competent" host cells), of which neutrophils and macrophages are the primary types in mammals.

Another engulfing process is endocytosis, by which intracellular pathogens exemplified by species of *Salmonella, Escherichia, Shigella, Helicobacter, Pseudomonas* and *Lactobacilli* gain entry to mammalian epithelial cells and replicate there. Two basic mechanisms in this regard are Clathrin-dependent receptor-mediated endocytosis, also known as "coated pit endocytosis" (Riezman, 1993), and Clathrin-independent endocytosis (Sandvig & Deurs, 1994). Either or both may be involved when an engulfing-competent cell that acts by endocytosis (*i.e*., an "endocytosis-competent" host cell) engulfs intact, recombinant minicells and expresses DNA carried by the minicells, in accordance with the invention. Representative endocytosis-competent cells are breast epithelial cells, enterocytes in the gastrointestinal tract, stomach epithelial cells, lung epithelial cells, and urinary tract and bladder epithelial cells.

The present invention is particularly useful for introducing, into engulfing-competent cells, nucleic acid molecules that, upon transcription and/or translation, function to ameliorate or otherwise treat a disease or modify a trait associated with a particular cell type, tissue, or organ of a subject. For purposes of the present description, these molecules are categorized as "therapeutic nucleic acid molecules."

For instance, transcription or translation of a given therapeutic nucleic acid molecule may be useful in treating cancer or an acquired disease, such as AIDS, pneumonia, emphysema, or in correcting inborn errors of metabolism, such as cystic fibrosis. Transcription or translation of a therapeutic nucleic acid may also effect contraceptive sterilization, including contraceptive sterilization of feral animals. Allergen-mediated and infectious agent-mediated inflammatory disorders also can be countered by administering, via the present invention, a therapeutic nucleic acid molecule that, upon expression in a patient, affects immune response(s) associated with the allergen and infectious agent, respectively. A therapeutic nucleic acid molecule also may have an expression product, or there may be a downstream product of post-translational modification of the expression product, that reduces the immunologic sequalae related to transplantation or that helps facilitate tissue growth and regeneration.

Alternatively, the expression product or a related, post-translational agent may be a protein, typified by erythropoietin, a growth factor such as TGA-β, a cytokine such as IL-2, IL-10, IL-12 or another of the interleukins, a serum leucoproteinase inhibitor, or an antibody such as CTLA4-Ig, that provides a desired benefit for the host. Other such proteins are, without limitation, α-, ß- and γ-globin, insulin, GM-CSF, M-CSF, G-CSF, EPO, TNF, MGF, adenosine deaminase, tumor-associated antigens such as viral, mutated or aberrantly-expressed antigens (CDK4, β-catenin, GnT-V, Casp8), cancer-specific antigens such as MAGE, BAGE, GAGE, PRAME, and NY-ESO-1, differentiation antigens such as tyrosinase, Melan-A/MART-1, gp100, and TRP-1/gp75, and overexpressed antigens such as Her2/neu and CEA. Further exemplars of the class of therapeutic nucleic acid molecules are DNAs coding for the cystic fibrosis transmembrane regulator (CFTR), Factor VIII or another blood protein, low density lipoprotein receptor, β-glucocerebrosidase, α- and β-galactosidase, insulin, parathyroid hormone, α-1-antitrypsin, *fas***R***,* and *fas***L***,* respectively.

In accordance with the present invention, expression of a therapeutic nucleic acid molecule by a host cell can supply a needed compound, mediate a targeted immune response, or interrupt a pathological process. For example, a therapeutic nucleic acid molecule can be implicated in an antisense or ribozyme therapy. In the present context, an antisense therapy involves introducing, pursuant to the invention, an antisense copy of a polynucleotide sequence, such that the RNA transcript of the copy hybridizes *in situ* with a messenger RNA (mRNA) transcript which corresponds to the polynucleotide sequence. This hybridization typically prevents the transcript from being translated into a protein or initiates a degradation pathway which destroys the mRNA.

The therapeutic nucleic acid also may encode short interfering RNA duplexes (siRNA's) within a mammalian cell. That is, siRNA's have been shown to suppress target genes in mammalian cells. This process of RNA interference (RNAi) is a method of sequence-specific, post-transcriptional gene silencing in animals, humans and plants and is initiated by double-stranded (ds) RNA that is homologous to the silenced gene. Viral-mediated delivery of siRNA to specifically reduce expression of targeted genes in various cell types has been successfully demonstrated (Haibin *et al.,* 2002).

For all of these and other diverse uses of a therapeutic nucleic acid molecule, the present description employs the rubric of "gene therapy," which also is connoted by the phrases "gene transfer," "gene delivery," and "gene-based vaccines," in relation to methodology or systems for transferring a therapeutic nucleic acid molecule into host cells, both *in vivo* and *ex vivo,* as described, for instance, in U.S. patent No. 5,399,346. Thus, a minicell-containing composition of the present invention can be used to achieve an *in situ* therapeutic effect, and for transforming cells outside of the body and then (re)introducing them to a subject. Pursuant to the present invention, the cells suitable for both *in vivo* and *ex vivo* approaches would be those that are engulfing-competent, such as phagocytes and epithelial cells.

As noted above, gene therapy may be effected to treat or prevent a genetic or acquired disease or condition. The therapeutic nucleic acid molecule encodes a product, such as functional RNA (*e.g.*, antisense or siRNA) or a peptide, polypeptide or protein, the *in vivo* production of which is desired. For example, the genetic material of interest can encode a hormone, receptor, enzyme, or (poly) peptide of therapeutic value. Such methods can result in transient expression of non-integrated transferred DNA, extrachromosomal replication and expression of transferred replicons such as episomes, or integration of transferred genetic material into the genomic DNA of host cells.

A therapeutic nucleic acid molecule may be the normal counterpart of a gene that expresses a protein that functions abnormally or that is present in abnormal levels in a disease state, as is the case, for example, with the cystic fibrosis transmembrane conductance regulator in cystic fibrosis (Kerem *et al.,* 1989; Riordan *et al.,* 1989; Rommens *et al.,* 1989), with ß-globin in sickle-cell anemia, and with any of α-globin, ß-globin and γ-globin in thalassemia. The therapeutic nucleic acid molecule can have an antisense RNA transcript or small interfering RNA, as mentioned above. Thus, an excess production of α-globin over ß-globin which characterizes ß-thalassemia can be ameliorated by gene therapy, in accordance with the present invention, using an intact minicell engineered to contain a plasmid incorporating a sequence that has an antisense RNA transcript vis-à-vis a target sequence of the α-globin mRNA.

In the treatment of cancer, a therapeutic nucleic acid molecule suitable for use according to the present invention could have a sequence that corresponds to or is derived from a gene that is associated with tumor suppression, such as the *p53* gene, the retinoblastoma gene, and the gene encoding tumor necrosis factor. A wide variety of solid tumors -- cancer, papillomas, and warts - should be treatable by this approach, pursuant to the invention. Representative cancers in this regard include colon carcinoma, prostate cancer, breast cancer, lung cancer, skin cancer, liver cancer, bone cancer, ovary cancer, pancreas cancer, brain cancer, head and neck cancer, and lymphoma. Illustrative papillomas are squamous cell papilloma, choroid plexus papilloma and laryngeal papilloma. Examples of wart conditions are genital warts, plantar warts, epidermodysplasia verruciformis, and malignant warts.

A therapeutic nucleic acid molecule for the present invention also can comprise a DNA segment coding for an enzyme that converts an inactive prodrug into one or more cytotoxic metabolites so that, upon *in vivo* introduction of the prodrug, the target cell in effect is compelled, perhaps with neighboring cells as well, to commit suicide. Preclinical and clinical applications of such a "suicide gene," which can be of non-human origin or human origin, are reviewed by Spencer (2000), Shangara *et al.* (2000) and Yazawa *et al.* (2002). Illustrative of suicide genes of non-human origin are those that code for HSV-thymidine kinase(*tk*), cytosine deaminase (CDA) + uracil phophoribosytransferase, xanthine-guanine phophoribosyl-transferase (GPT), nitroreductase (NTR), purine nucleoside phophrylase (PNP, DeoD), cytochrome P450 (CYP4B1), carboxypeptidase G2 (CPG2), and D-amino acid oxidase (DAAO), respectively. Human-origin suicide genes are exemplified by genes that encode carboxypeptidase A1 (CPA), deoxycytidine kinase (dCK), cytochrome P450 (CYP2B1,6), LNGFR/FKBP/Fas, FKBP/Caspases, and ER/p53, respectively.

A suicide-gene therapy according to the present invention could be applied to the treatment of AIDS. This strategy has been tested with suicide vectors that express a toxic gene product as soon as treated mammalian cells become infected by HIV-1. These vectors use the HIV-1 regulatory elements, Tat and/or Rev, to induce the expression of a toxic gene such as α-diphtheria toxin, cytosine deaminase, or interferon-a2 after infection by HIV-1 (Curiel *et al.,* 1993; Dinges *et al.,* 1995; Harrison *et al.,* 1992a; Harrison *et al.,* 1992b; Ragheb *et al.,* 1999). Cells could be transduced by these vectors, using the recombinant-minicell approach of this invention, and would be eliminated faster than untransduced cells after HIV infection, preventing viral replication at the expense of cell death.

A nucleic acid molecule to be introduced via the approach of the present invention can include a reporter element. A reporter element confers on its recombinant host a readily detectable phenotype or characteristic, typically by encoding a polypeptide, not otherwise produced by the host, that can be detected, upon expression, by histological or *in situ* analysis, such as by *in vivo* imaging techniques. For example, a reporter element delivered by an intact minicell, according to the present invention, could code for a protein that produces, in the engulfing host cell, a colorimetric or fluorometric change that is detectable by *in situ* analysis and that is a quantitative or semi-quantitative function of transcriptional activation. Illustrative of these proteins are esterases, phosphatases, proteases and other enzymes, the activity of which generates a detectable chromophore or fluorophore.

Preferred examples are *E. coli* β-galactosidase, which effects a color change via cleavage of an indigogenic substrate, indolyl-β-D-galactoside, and a luciferase, which oxidizes a long-chain aldehyde (bacterial luciferase) or a heterocyclic carboxylic acid (luciferin), with the concomitant release of light. Also useful in this context is a reporter element that encodes the green fluorescent protein (GFP) of the jellyfish, *Aequorea victoria,* as described by Prasher *et al.* (1995). The field of GFP-related technology is illustrated by two published PCT applications, WO 095/21191 (discloses a polynucleotide sequence encoding a 238 amino-acid GFP apoprotein, containing a chromophore formed from amino acids 65 through 67) and WO 095/21191 (discloses a modification of the cDNA for the apopeptide of *A*. *victoria* GFP, providing a peptide having altered fluorescent properties), and by a report of Heim *et al.* (1994) of a mutant GFP, characterized by a 4-to-6-fold improvement in excitation amplitude.

Another type of a reporter element is associated with an expression product that renders the recombinant minicell resistant to a toxin. For instance, the *neo* gene protects a host against toxic levels of the antibiotic G418, while a gene encoding dihydrofolate reductase confers resistance to methotrexate, and the chloramphenicol acetyltransferase (CAT) gene bestows resistance to chloramphenicol.

Other genes for use as a reporter element include those that can transform a host minicell to express distinguishing cell-surface antigens, *e.g.*, viral envelope proteins such as HIV gp120 or herpes gD, which are readily detectable by immunoassays.

A nucleic acid molecule to be introduced via the approach of the present invention also can have a desired encoding segment linked operatively to a regulatory element, such as a promoter, a terminator, an enhancer and/or a signal sequence. A suitable promoter can be tissue-specific or even tumor-specific, as the therapeutic context dictates.

A promoter is "tissue-specific" when it is activated preferentially in a given tissue and, hence, is effective in driving expression, in the target tissue, of an operably linked structural sequence. The category of tissue-specific promoters includes, for example: the hepatocyte-specific promoter for albumin and a₁-antitrypsin, respectively; the elastase I gene control region, which is active in pancreatic acinar cells; the insulin gene control region, active in pancreatic beta cells; the mouse mammary tumor virus control region, which is active in testicular, breast, lymphoid and mast cells; the myelin basic protein gene control region, active in oligodendrocyte cells in the brain; and the gonadotropic releasing hormone gene control region, which is active in cells of the hypothalamus. See Frain *et al.* (1990), Ciliberto *et al.* (1985), Pinkert *et al.,* (1987), Kelsey *et al.* (1987), Swift *et al*. (1984), MacDonald (1987), Hanahan, (1985), Leder *et al.* (1986), Readhead *et al*. (1987), and Mason *et al.* (1986).

There also are promoters that are expressed preferentially in certain tumor cells or in tumor cells *per se,* and that are useful for treating different cancers in accordance with the present invention. The class of promoters that are specific for cancer cells is illustrated by: the tyrosinase promoter, to target melanomas; the MUC1/Df3 promoter, to target breast carcinoma; the hybrid *myo*D enhancer/SV40 promoter, which targets expression to rhabdomyosarcoma (RMS); the carcinoembryonic antigen (CEA) promoter, which is specific for CEA-expressing cells such as colon cancer cells, and the hexokinase type II gene promoter, to target non-small cell lung carcinomas. See Hart (1996), Morton & Potter (1998), Kurane *et al.* (1998) and Katabi *et al.* (1999).

A signal sequence can be used, according to the present invention, to effect secretion of an expression product or localization of an expression product to a particular cellular compartment. Thus, a therapeutic polynucleotide molecule that is delivered via intact minicells may include a signal sequence, in proper reading frame, such that the expression product of interest is secreted by an engulfing cell or its progeny, thereby to influence surrounding cells, in keeping with the chosen treatment paradigm. Illustrative signal sequences include the haemolysin C-terminal secretion sequence, described in U.S. patent No. 5,143,830, the BAR1 secretion sequence, disclosed in U.S. patent No. 5,037,743, and the signal sequence portion of the zsig32 polypeptide, described in U.S. patent No. 6,025,197.

The ability of intact, recombinant minicells of the invention to maintain integrity *in vivo* makes possible their use in gene therapy, as described above. Intact minicells also carry none of the bacterial genomic DNA that is present in recombinant bacterial cells, for example, of *Shigella flexneri, Listeria monocytogenes, Escherichia coli* or *Salmonella typhimurium,* which others have used to transfer eukaryotic expression plasmids into host cells. See *Sizemore et al.* (1995); Gentschev *et al.* (2000); Catic *et al.* (1999); Dietrich *et al.* (1998); and Courvalin *et al.* (1995). Accordingly, gene therapy with intact minicells, pursuant to the present invention, does not entail the risk, associated with use of the recombinant bacteria, of the unintended transfer of a bacterial or an antibiotic resistance-marker gene to microbial flora which are indigenous to the patient. Furthermore, recombinant bacteria must be cleared from the patient by means of cell-mediated immunity and, hence, are unsuitable for gene therapy of an immunocompromised patient suffering, for example, from cancer or AIDS.

Minicells can be prepared from any bacterial cell of Gram-positive or Gram-negative origin. Because the present invention employs intact minicells, rather than minicell protoplasts, the present invention does not require and preferably does not involve any antibiotic or chemical pretreatment of minicells.

A minicell-producing bacterial strain can be generated by mutation of a *min* gene, for example, through a partial deletion of a *min*CDE gene sequence, as illustrated in Examples 1 and 2 below. To obtain recombinant, intact minicells, according to the invention, bacterial cells of the chosen minicell-producing strain are transformed via a standard technique, including but not limited to the use of electroporation (Shigekawa & Dower, 1988), chemical methodology (Hanahan, 1983), shuttle vectors (Marcil & Higgins, 1992), and conjugation (Firth *et al.,* 1996) or transduction (Davis *et al.,* 1980). For this transformation, a therapeutic nucleic acid molecule from any eukaryotic, prokaryotic or synthetic source is inserted into a suitable, commercially available or end user-proprietary plasmid vector. A selected DNA can be operably linked to the control elements required for gene delivery and/or expression of the DNA in the cells that engulf the recombinant minicells *in vivo.*

Recombinant minicells preferably are analyzed *in vitro* to ensure that they can deliver the recombinant plasmid or DNA sequences to the target cell nucleus, and that recombinant gene expression occurs in the target cell. The assay for expression will depend upon the nature of the heterologous gene. Expression may be monitored by a variety of methods, including immunological, histochemical or activity assays. The expression of a fluorescent marker gene can be visualized microscopically, and this provides a particularly convenient assay. For example, the gene encoding green fluorescence protein under the control of a eukaryotic gene expression promoter, such as the CMV promoter, may be transferred on a plasmid by recombinant minicells to target eukaryotic cells (see below). Additionally, northern blot analysis or Reverse Transcriptase PCR (RT-PCR) may be used to assess transcription using appropriate DNA or RNA probes. If antibodies to the polypeptide encoded by the heterologous gene are available, Western blot analysis, immunohistochemistry or other immunological techniques can be used to assess the production of the polypeptide. Appropriate biochemical assays also can be used if the heterologous gene is an enzyme. For instance, if the heterologous gene encodes antibiotic resistance, then a determination of the resistance of infected cells to the antibiotic can be used to evaluate expression of the antibiotic resistance gene.

The engulfing-competency of a putative host ("target") cell can be evaluated by testing the efficacy of DNA delivery in culture. Thus, a targeted tumor could be subjected to biopsy, and the resultant tissue sample would be used, in conventional manner, to obtain representative tumor cells in culture, to test for an ability to engulf recombinant minicells of the present invention that carry, for example, a suitable reporter element. Additionally or alternatively to testing such a primary cell culture, one may gauge the capacity to engulf minicells by testing a cell line that is representative of the type of tissue to which the therapeutic protocol is keyed, pursuant to the present invention. Cell culture techniques are well documented, for instance, by Freshner (1992).

In accordance with the invention, recombinant minicells can be purified from parent cells by several means. One approach utilizes sucrose gradient methodology described, for example, by Reeve (1979) and by Clark-Curtiss *et al.* (1983), followed by treatment with an antibiotic, such as gentamycin (200 µg/ml, 2 hours), to kill residual live bacteria.

With the conventional methodology, the purity achieved is one contaminating parent cell per 10⁶ to 10⁷ minicells, at best. For *in vivo* applications, according to the present invention, doses greater than 10⁶ may be required and may be as high as 10¹⁰ per dose, which, with the aforementioned contamination ratio, would translate into 10,000 live parent cells per dose. Such a contamination level could be fatal, particularly in immuno-compromised subjects.

In addition, the conventional technology employs media that contain a gradient-formation agent, such as sucrose, glycerol or Percoll^{®}, the presence of which is undesirable for *in vivo* uses, as presently contemplated. Thus, the toxicity of Percoll^{®} restricts it to "research purposes only" contexts, while sucrose for a gradient imparts a high osmolarity that can cause physiological changes in the minicells.

For gene therapy applications according to the present invention, therefore, it is preferable to minimize parent cell contamination and to utilize media that are more biologically compatible. To achieve these goals, the present inventors have found it unexpectedly advantageous to combine cross-flow filtration (feed flow is parallel to a membrane surface) and dead-end filtration (feed flow is perpendicular to the membrane surface). Generally, see Forbes (1987). Optionally, this combination can be preceded by a differential centrifugation, at low centrifugal force, to remove some portion of the bacterial cells and thereby enrich the supernatant for minicells. Also optionally, the combination can be followed by an antibiotic treatment to kill residual parent bacterial cells.

Cross-flow filtration, depending on the filter pore size, can separate minicells from larger contaminants such as parent bacterial cells, and from smaller contaminants such as bacterial blebs, free endotoxin, nucleic acids cellular debris and excess liquid. To separate minicells from larger contaminants, the nominal pore size of cross-flow filters should allow minicells to permeate through the filters, but not large bacterial cells. A 0.45 µm pore size is preferred for this purpose because minicells are approximately 0.4 µm in diameter, whilst bacterial cells are larger. To separate minicells from smaller contaminants, the nominal pore size of cross-flow filters should allow smaller contaminants to permeate through the filters, but not minicells. A 0.2 µm pore size is preferred for this purpose because bacterial blebs range in diameter from 0.05 µm to 0.2 µm, and the other smaller contaminants are less than 0.2 µm.

Effective application of cross-flow filtration in this context typically entails at least one step involving a larger pore size, around 0.45 µm, followed by at least one step with a smaller pore size, around 0.2 µm. Between or during serial cross-flow filtration steps, diafiltration may be performed to maximize recovery of minicells.

The use of cross-flow filtration accommodates suspensions carrying heavy loads of particulate matter, such as bacterial cultures, which may carry loads of 10¹¹ to 10¹³ bacterial and minicell populations per liter of culture. To minimize filter fouling and the consequent loss of minicells, the bacterial/minicell culture may be diluted, preferably 5-fold to 10-fold. Dilutions also permit use of appropriately low atmospheric pressure and flow rate.

To remove residual parent bacterial cells remaining after cross-flow filtration, dead-end filtration is performed. For this purpose, the use of at least one dead-end filtration, employing a pore size of about 0.45 µm, is preferred.

Generally, filtration provides a sterile preparation of minicells suitable for gene transfer studies. For *in vivo* gene transfer, an antibiotic treatment is preferably performed further to reduce the risks from bacterial cell contamination. For instance, minicells may be resuspended in growth medium that contains an antibiotic to which the parent bacterial strain is sensitive. The appropriate amount of a given antibiotic for this purpose can be determined in advance by conventional techniques.

A serial cross-flow filtration/dead-end filtration arrangement, as described, not only dispenses with the use of gradient-formation agents but also provides for a purity that exceeds 10⁻⁷ (*i.e.,* fewer than one parent cell per 10⁷ minicells). Preferably, the purity exceeds 10⁻⁸ and, more preferably, is on the order of about 10⁻⁹. The serial cross-flow filtration/dead-end filtration arrangement also provides for better quality control. In addition, there is no need for an ampicillin-, cycloserine-, or other antibiotic-resistance gene, as required by the technique of Clarke-Curtiss and Curtiss (1983). Furthermore, the serial purification approach employs no DNA-damaging radiation, in contrast to a methodology described Sancar *et al.* (1979); hence, a therapeutic nucleic acid molecule delivered with minicells prepared via a serial cross-flow filtration/dead-end filtration arrangement can be free of radiation-induced, non-specific mutation.

A composition consisting essentially of recombinant minicells of the present invention (that is, a composition that includes such minicells with other constituents that do not interfere unduly with the DNA-delivering quality of the composition) can be formulated in conventional manner, using one or more physiologically acceptable carriers or excipients. Formulations for injection may be presented in unit dosage form, *e.g.*, in ampules or vials, or in multi-dose containers, with or without an added preservative. The formulation can be a solution, a suspension, or an emulsion in oily or aqueous vehicles, and may contain formulatory agents, such as suspending, stabilizing and/or dispersing agents. A suitable solution is isotonic with the blood of the recipient and is illustrated by saline, Ringer's solution, and dextrose solution. Alternatively, minicells may be in lyophilized powder form, for reconstitution with a suitable vehicle, *e.g.*, sterile, pyrogen-free water or physiological saline. Minicells also may be formulated as a depot preparation. Such long-acting formulations may be administered by implantation (for example, subcutaneously or intramuscularly) or by intramuscular injection.

A minicell-containing composition of the present invention can be administered via various routes and to various sites in a mammalian body, to achieve the therapeutic effect(s) desired, either locally or systemically. Delivery may be accomplished, for example, by oral administration, by application of the formulation to a body cavity, by inhalation or insufflation, or by parenteral, intramuscular, intravenous, intraportal, intrahepatic, peritoneal, subcutaneous, intratumoral, or intradermal administration.

The nature of the application contemplated likewise will influence (or be influenced by) the choice of bacterial source for the recombinant minicells employed to deliver a therapeutic nucleic acid molecule. For example, *Salmonella, Escherichia* and *Shigella* species carry adhesins that are recognized by endocytosis-mediating receptors on enterocytes in the gastrointestinal tract may be suitable for oral administration, to deliver a therapeutic nucleic acid molecule that is effective for colon cancer cells. Similarly, minicells derived from *Helicobacter pylori,* carrying adhesins specific for stomach epithelial cells, could be suited for oral delivery aimed at stomach cancer cells. Inhalation or insufflation may be ideal for administering intact minicells derived from a *Pseudomonas* species that carry adhesins recognized by receptors on lung epithelial cells; this, for delivery, to the lungs of a cystic fibrosis patient, of a therapeutic nucleic acid molecule encoding CFTR protein, for example. Minicells derived from *Lactobacilli* bacteria, which carry adhesins specific for urinary tract and bladder epithelial cells, could be well-suited for intraurethral delivery of a therapeutic nucleic acid molecule to a urinary tract and or a bladder cancer.

The present invention can be used to deliver a range of nucleic acid molecules, which can be cDNA as well as genomic DNA or RNA, and can be in the sense or the anti-sense orientation. The nucleic acid molecule present in an intact minicell, pursuant to the present invention, can take the form of a plasmid, expression vector, or other genetic construct, but is not genomic DNA originating from the bacterial cell that gave rise to the minicell. Suitable for use in the present invention is any desired DNA or RNA sequence from a eukaryotic, prokaryotic, or synthetic source which may be placed under the translational and transcriptional control of a eukaryotic gene expression promoter, or which may be expressed in the mammalian cell using trans-activating factors from the host cell.

### Example 1. Generation of bacterial minicells from Gram-negative bacteria, Salmonella typhimurium, Escherichia coli and Shigella flexneri

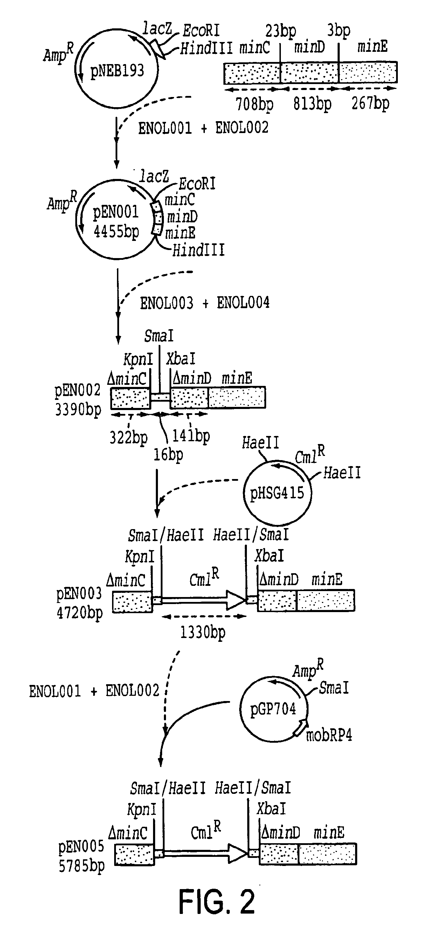

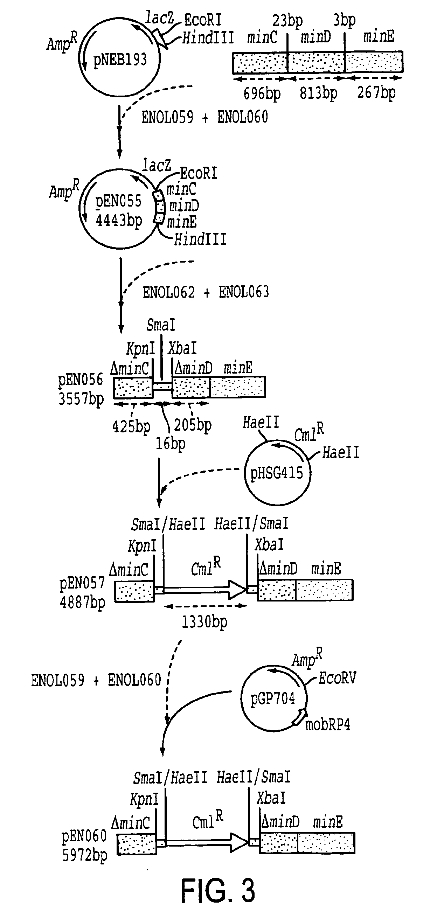

Minicell-producing bacterial strains from Gram-negative bacteria were generated, as described here (A, B and C) and illustrated in Figures 2 and 3.

### General Materials and Methods

The bacterial strains used in the instances below are listed and referenced in Table 1. All bacteria were grown from glycerol stocks maintained at -80°C. *Salmonella, E. coli, Shigella and Listeria* strains were grown in Trypticase Soy Broth (TSB) (BBL brand purchased from Bacto Labs, Liverpool, NSW, Australia). It was prepared according to the manufacturer's instructions at 30 gm/l, and autoclaved at 121°C for 15 minutes. Liquid culture was grown in a shaking incubator at 37°C. *Shigella* strains were differentiated from *E*. *coli* by plating on XLD agar (Xylose-Lysine-Desoxycholate Agar) plates to result in red and yellow colonies respectively. XLD was purchased from Oxoid (Melbourne, Australia). It was prepared according to the manufacturer's instructions at 53 gm/l, then boiled for 2 minutes. Antibiotics were used at the following concentrations in liquid and solid media: ampicillin, 100 µg/ml, chloramphenicol, 30 µg/ml, Kanamycin, 30 µg/ml.

**Table 1. Bacterial strains used**

| ***E. coli* strain** | **Genotype / relevant characteristics** | **Reference** |
|---|---|---|
| ENIh001 | SM100pir; F- *sup*E44, *thi*-1, *thr*-1, *leu*B6, *lac*Y1, *ton*A21, *rec*A:RP4-2-Tc::Mu lambdapir, Tn*pho*A, *ori*R6K, tra- mob+ | Miller and Mekalanos (1988). |
| ENE105 | Strain ENIh001 carrying plasmid pEN060 (Fig. 3) | The present disclosure |
| ENIh003 | JM109; F' *tra*D36 *pro*A+ *pro*B+ *lac*l^{q} *lac*ZDM15/*rec*A1 *end*A1 gyrA96 (Nal^{r} *thi hsd*R17 *sup*E44 *rel*A1 D(*lac-pro*AB) *mcr*A | Yanisch-Perron *et al.* (1985) |

| ***Salmonella* strain** | | |
|---|---|---|
| ENIh007 | *Salmonella enterica* serovar Typhimurium. Clinical isolate from sheep. | Institute of Medical and Veterinary Services, Adelaide, SA, Australia. Reference strain J98/00413 |
| ENSm083 | *Salmonella choleraesuis* subsp. *choleraesuis* (Smith) Weldin serotype Typhimurium deposited as *Salmonella typhimurium* | ATCC 14028 |
| SL3261 | *Salmonella typhimurium aroA-* | Hoiseth *et al.,* (1981) |
| SL5283 | *Salmonella typhimurium hsd*R*-, hsd*M+ | Hoiseth *et al.,* (1981) |

| ***Shigella* strain** | | |
|---|---|---|
| ENSf001 | S. *flexneri* serotype 2b | ATCC 12022 |

| ***Listeria* strain** | | |
|---|---|---|
| ENLM001 | *Listeria monocytogenes* Gibson ATCC 7644 | J. Pathol. Bacteriol. 45: 523, (1937) |

Where required, strains were grown in M9 minimal medium supplemented with 1% glucose. Additional supplements were added depending on the strain. For *E. coli* strain ENIh003, 1 mM Thiamine was added. For *S*. *flexneri* strain ENSf001, M9 was supplemented with 0.4 mM Serine, 0.2 mM Proline, 0.001% Phenylalanine, 0.001% Tryptophan, 0.001% Tyrosine, 0.001% Histidine, 0.002 mg/ml Valine, 0.0125 mg/ml Nicotinic acid, 0.001% Thiamine, and 0.0225 mg/ml Methionine.

Plasmid DNA was purified using the Qiaprep Spin Miniprep Kit (Qiagen Inc., Victoria, Australia). Genomic DNA for *Salmonella, Shigella* and *E. coli* strains was prepared using the basic protocol from Current Protocols in Molecular Biology (Chapter 2, Section I, Unit 2.4; John Wiley & Sons, Inc.). All restriction and modification enzymes used were purchased from Promega (Madison, WI, USA) or Roche (Castle Hill, NSW, Australia), except for Deep Vent DNA Polymerase, which was purchased from New England Biolabs (Beverly, MA, USA).

Genomic DNA of *L. monocytogenes* (ENLM001) was purified by conventional methods (Ausubel et. al., Current Protocols in Molecular Biology, John Wiley and Sons Inc.) with modifications as described. A 1.5 ml sample of an overnight culture was centrifuged at 13,200 rpm for 3 minutes and the supernatant was discarded. The bacterial cell pellet was resuspended in 1 ml of TE buffer (10mM Tris pH 8.0; 1 mM EDTA pH 8.0) with 2.5 mg/ml lysozyme, and incubated at 37°C for 1 hour. RNAse A was then added to a final concentration of 15 mg/ml, and the solution was incubated at room temperature for 1 hour. Then, 100 µg/ml Proteinase K and 0.5% SDS were added and the mixture was further incubated for an hour at 37°C. Subsequently, 200 µl of 5M NaCl was mixed thoroughly into the solution, followed by 160 µl of CTAB/NaCl solution (10% CTAB in 0.7 M NaCl). This mixture was then incubated for 10 minutes at 65 °C. The sample was extracted with an equal volume of chloroform/isoamyl alcohol followed by an extraction with an equal volume of phenol/chloroform/isoamyl alcohol. Genomic DNA was precipitated from solution with 0.6 volume of isopropanol and 1/10^{th} volume of 5M sodium acetate. The DNA pellet was washed with ethanol and air dried before resuspension in TE buffer.

PCR primers were synthesized and purchased from Sigma-Genosys (Castle Hill, NSW, Australia). The basic PCR protocol followed was as follows. Reaction components for all 50 µl PCR included 1X buffer, 200 µM dNTPs, 50 pmol each primer, 1 Unit of Deep Vent DNA polymerase, 50 pmol of genomic DNA template (25 pmol for plasmids), nuclease-free water to 50 µl, with PCR performed in 0.2 ml tubes in a Gradient PCR Express Thermalcycler from Thermo Hybaid (Ashford, Middlesex, UK). PCR conditions to obtain the *min*CDE gene cluster were as follows: 94°C for 4 minutes; followed by 30 cycles at 94°C for 35 seconds, 60°C for 30 seconds, 72°C for 2.5 minutes; followed by a final cycle at 94°C for 35 seconds, 60°C for 35 seconds, 72°C for 5 minutes. PCR conditions to obtain the Δ*min*CDE cassette were as follows: 94°C for 4 minutes; followed by 30 cycles at 94°C for 35 seconds, 60°C for 30 seconds, 72°C for 2 minutes; followed by a final cycle at 94°C for 35 seconds, 60°C for 35 seconds, 72°C for 4 minutes. PCR conditions to obtain the Δ*min*CDE::*Cml* cassette were as follows: 94°C for 4 minutes; followed by 30 cycles at 94°C for 35 seconds, 60°C for 30 seconds, 72°C for 3 minutes; followed by a final cycle at 94°C for 35 seconds, 60°C for 35 seconds, 72°C for 6 minutes.

The standard molecular biology protocols followed were as described in Sambrook et al. (1989) and in CURRENT PROTOCOLS IN MOLECULAR BIOLOGY (John Wiley & Sons, Inc., NJ, USA).

The minicell yield was determined microscopically, using a Leica Model DMLB light microscope with image analysis by means of a Leica DC camera and Leica IM image management software. Samples were viewed using Darkfield microscopy at 40X or oil immersion at 100X magnification. Coverslips on glass slides were sealed with 1.5% agarose. Purity of each batch of minicells was determined by plating 10% of the volume on Trypticase Soy Agar plates and incubation overnight at 37°C. The method routinely provided a very high purity with one contaminating cell in 10⁹ minicells. The minicell suspensions were resuspended in TSB with the appropriate antibiotics to which the parent bacteria were determined to be sensitive and the cultures were incubated with shaking at 37°C for 4 hours, to kill all residual parent bacteria. For example, the S. *typhimurium min*CDE- strain was determined to be sensitive to Ampicillin and hence the minicell suspension was incubated in TSB containing 50 µg/ml of Ampicillin for 4 hours, to ensure that if there were any residual bacteria, then they would be killed. The minicells then were collected by centrifugation at 10,000g for 30 minutes, washed four times in 1 x BSG buffer (to eliminate the growth medium), and resuspended in the desired volume for down-stream experiments.

### [A] Generation of minicell producing strains from two different strains of Salmonella typhimurium.

A schematic diagram of plasmid construction is shown in Figure 2. Bacterial strains, plasmids and PCR primers used are shown in Tables 1, 2 and 3 respectively.

The *E. coli min*CDE gene sequences, see Mori (1996), were used to search the Genbank database for homologous DNA sequences using the method of FASTA analysis (Pearson and Lipman, 1988). The TYPN3 contig 101 DNA sequence from the *S*. *typhi* genome (CT18) was found to be homologous to the *E. coli min*CDE sequences. The oligonucleotide primers ENOL001 and ENOL002 were designed on the basis of these data and were used to prime the synthesis of the intact *min*CDE genes from *S*. *typhimurium* strain ENIh008 as an *Eco*RI*-min*CDE-*Hind*III fragment.

This fragment was cloned into the *Eco*RI/*Hind*III sites of pNEB193 to create a plasmid designated as pEN001, which was propagated in *E. coli* strain JM109 to result in strain ENE001. Primers ENOL003 and ENOL004 were designed to delete a total of 1081 bp of sequence from the *min*CDE cassette in pEN001, while simultaneously inserting 16 base pairs (bp) containing the unique *KpnI, Sma*I and *Xba*I restriction sites as locations for future insertion of one or more marker genes.

The deleted sequence included 386 base pairs from the 3' end of the *min*C gene, the 23-base pair intervening sequence upstream of the *min*D gene and 672 base pairs from the 5' end of the *min*D gene. This resulted in the Δ*min*CDE deletion cassette (755 base pairs) in plasmid pEN002 harbored in strain ENE003. The chloramphenicol resistance gene from pHSG415 (Fig. 2, Table 2), 1330-base pair *Hae*II fragment/blunt-ended) was cloned into the *Sma*I site of pEN002 to obtain plasmid pEN003, carrying the Δ*min*CDE::*Cml*^{R} deletion cassette with the *Cml*^{R} gene cloned in the clockwise orientation. This was designated strain ENE006.

**Table 2. Plasmids used in this study.**

| **Plasmid** | **Relevant characteristics** | **Reference** |
|---|---|---|
| pHSG415 | Low-copy number, temperature sensitive, mobilisable | Hashimotoh-Gotoh *et al.,* (1981) |
| pGP704 | Derivative of pBR322 where the *oriE1* has been replaced with *oriR6K.* The R6K origin of replication requires for its function a protein called pi, encoded by the *pir* gene. In *E. coli* the pi protein can be supplied in *trans* by a prophage *(*□*pir)* that carries a cloned copy of the *pir* gene. The plasmid also contains a 1.9-kb *Bam*HI fragment encoding the *mob* region of RP4. Thus, pGP704 can be mobilized into recipient strains by transfer functions provided by a derivative of RP4 integrated in the chromosome of *E. coli* strain SM10. However, once transferred it is unable to replicate in recipients that lack the pi protein. Amp^{R} | Miller and Mekalanos (1988) |
| pNEB193 | It is a pUC 19 derivative that carries single sites for unique 8-base cutters: *Asc I, Pac* I and *Pme* I. The polylinker carries the unique restriction sites *Eco*RI*, Sac*I, *Kpn*I*, Sma*I*, Asc*I, *Bss*HII, *Bam*HI*, Pac*I, *Xba*I*, Sal*I*, Pme*I*, Sbf*I*, Pst*I*, Sph*I, *Hind*III. The polylinker does not interrupt the *lac*Z reading frame. Amp^{R} | Purchased from New England Biolabs, Inc. Beverly, MA, USA |
| pMK4 (5.6 kb) | *E. coli, Staphylococcus aureus, Bacillus subtilis* shuttle vector, lacZ' (from pUC9), Cml^{R} (from pC194) in *Bacillus,* Amp^{R} in *E*. *coli.* Cml^{R} in Bacillus | Sullivan *et al.,* (1984) |
| pVA838 (9.2 kb) | *E. coli* and *Streptococcus sanguis* shuttle vector, Erythromycin resistant (Em^{R}) in *S*. *sanguis,* Cml^{R} in *E*. *coli.* | Macrina *et. al.,* (1982) |
| pRB373 (5.8 kb) | *E. coli* and *B. subtilis* shuttle vector, *bla* (Amp^{R}) and *E*. *coli ori* were derived from pBR322. Kanamycin resistance (Km^{R}), Belomycin resistance (Bm^{R}) and Gram-positive *ori* were derived from pUB110. *to* (transcription terminator) is from phage λ. | Brückner (1992) |
| pEGFP | Carries a red-shifted variant of wild-type green fluorescent protein (GFP) which has been optimized for brighter fluorescence and higher expression in mammalian cells. (Excitation maximum = 488 nm; emission maximum = 507 nm.) Upstream sequences flanking EGFP have been converted to a Kozak consensus translation initiation site to further increase the translation efficiency in eukaryotic cells. The EGFP gene was cloned between the two MCS of the pUC19 derivative pPD16.43. The EGFP gene was inserted in frame with the *lacZ* initiation codon from pUC 19 so that a EGFP fusion protein is expressed from the *lac* promoter in *E*. *coli.* The pUC backbone of EGFP provides a high-copy-number origin of replication and an ampicillin resistance gene for propagation and selection in *E*. *coli.* | Purchased from Clontech Laboratories, Palo Alto, CA, USA. |
| pEGFP-C | EGFP is as described for plasmid pEGFP. Sequences flanking EGFP have been converted to a Kozak consensus translation initiation site to further increase the translation efficiency in eukaryotic cells. The EGFP gene is expressed from the human cytomegalovirus immediate early promoter and hence the plasmid only expresses EGFP in mammalian calls and not in bacterial cells. The MCS in pEGFP-C1 is between the EGFP coding sequences and the SV40 poly A (downstream of the EGFP gene) which directs proper processing of the 3' end of the EGFP mRNA. The vector backbone also contains an SV40 origin for replication in mammalian cells expressing the SV40 T-antigen. A neomycin resistance cassette (neo^{r}), consisting of the SV40 early promoter, the neomycin/kanamycin resistance gene of Tn5, and polyadenylation signals from the Herpes simplex thymidine kinase (HSV TK) gene, allows stably transfected eukaryotic cells to be selected using G418. A bacterial promoter upstream of this cassette expresses kanamycin resistance in *E*. *coli.* The pEGFP-C1 backbone also provides a pUC origin of replication for propagation in *E*. *coli* and an f1 origin for single-stranded DNA production. | Purchased from Clontech Laboratories, Palo Alto, CA, USA. |

The Δ*min*CDE::*Cml*^{R} deletion cassette was amplified from plasmid pEN003 by polymerase chain reaction (PCR) using primers ENOL001 and ENOL002, blunt-ended and cloned into the *Sma*I site of suicide plasmid pGP704 (Fig. 2; Table 2). The plasmid, designated pEN005, was transformed into strain ENIh001 (SM10λpir; Table 1) to give strain number ENE007. Strain ENE007 was used as the donor strain in a conjugation (filter mating) experiment, with *S*. *typhimurium* strains ENIh007 and ENSm083 (Table 1) as the recipient. Overnight, static cultures of the donor and recipient were grown in TSB at 37°C. Cultures were mixed in a filter mating conjugation on Hybond N⁺ membranes on TSA plates at a donor:recipient ratio of 1:3 and incubated at 37°C for 8 hours. The cells were recovered and washed twice in a sterile saline solution. The cell pellet was resuspended in saline and plated on 150 mm Petri plates. The plates were incubated for up to 72 hours at 37°C.

The ex-conjugants were selected on M9 minimal medium with 1.5 % glucose and 30 µg/ml chloramphenicol. The donor strain is counter-selected under these conditions, due to extra auxotrophic requirements, whilst the recipient strain cannot grow, due to its chloramphenicol sensitivity. Therefore this experiment selected for *S. typhimurium* ex-conjugants carrying the plasmid-encoded chloramphenicol resistance. The colonies were screened for the desired phenotype of Cml^{R} and Amp^{s} by patching isolates on to M9 minimal medium containing ampicillin (Amp) or chloramphenicol (Cml). From 79 isolates of the ENE007 x ENIh007 conjugation that exhibited Cml^{R}, a total of 18 isolates were found to be Amp^{s}. Similarly, from 56 isolates of the ENE007 x ENSm083 conjugation, 19 were found to be Amp^{s}.

To determine whether the isolates were chromosomally deleted for the *minCD* genes, overnight cultures were visualized via darkfield microscopy at 40X magnification. Minicells were visualized in the mixed culture for all 27 isolates. All isolates showed the presence of minicells while the parent control strains were absent for minicells. Purified minicells were tested for agglutination with 4-0 *Salmonella* Somatic Agglutinating Serum (rabbit) ZC13 (Murex Diagnostics, Norcross, Georgia USA).

The recombinant bacterial strain was grown under laboratory conditions that are optimal for production of recombinant minicells. Bacterial growth is accomplished using standard bacteriological media, such as those described in Sambrook *et al.* (1989), and using optimal growth conditions, which can be readily determined by conventional technique.

### [B] Generation of minicell producing strain from Shigella flexneri.

Figure 3 depicts the relevant steps in the genetic construction of a minicell-producing strain from *S*. *flexneri* serotype 2b. The cloning protocol was similar to that followed for construction of minicell-producing *S*. *typhimurium* strains, detailed above.

To clone the *min*CDE gene cluster from *S. flexneri* serotype 2b (Table 1), PCR cloning primers were designed based on a database search of the in-progress sequencing project for the complete *Shigella flexneri,* serotype 2a genome sequence. PCR primers ENOL059 and ENOL060 carrying *Eco*RI and *Hind*III tails respectively (Table 3) were used to amplify the 1808 bp *min*CDE gene cluster from genomic DNA purified from *Shigella flexneri,* serotype 2b. The amplified fragment was cloned into the *Eco*RI and *Hind*III sites of plasmid pNEB193 (Table 2), resulting in plasmid pEN055. The insert DNA was sequenced and confirmed to be the *min*CDE DNA from the parent *Shigella* bacterium.

**Table 3. Oligonucleotides used in this study. Restriction enzyme sites and DNA sequence characteristics are shown in brackets preceding the respective DNA segment. (F) and (R) represent forward and reverse PCR primers respectively.**

| **Oligo-nucleotide** | **Sequence** | **Product** |
|---|---|---|
| ENOL001 | *5' (**Tail**)* CTC TCA CTG *(****EcoR*I***)* GAA TTC *(****min*C***)* ATG TCA AAC ACG CCA ATC GAG C 3' | *(F) S. typhimurium minCDE* |
| ENOL002 | *5' (**Tail)*** CTC CTG GCA **(*Hind*III)** AAG CTT **(*min*E)** TTA TTT TGA CTC TTC GGC TTC CG 3' | (R) *S. typhimurium minCDE* |
| ENOL003 | 5' ***(Tail)*** CTC TGC TAG TCA **(*Sma*I*-Kpn*I)** CCC GGG TAC C **(*min*C)** GCC GAA CCG CTT TCT CTT TAC C 3' | (R) *S. typhimurium* deletion of minCDE to get Δ*min*CDE |
| ENOL004 | *5' **(Tail)*** CTC TGC TAG TCA **(*Sma*I)** CCC GGG **(*Xba*I)** TCT AGA **(*min*D)** GAA CCG GTG ATT CTT GAC GCC A 3' | *(F) S.typhimurium* deletion of minCDE to get Δ*min*CDE |
| ENROL059 | *5' **(Tail)*** CTC TCA CT ***(Eco*RI*)*** GAA TTC ***(min*C*)*** ATG TCA AAC ACT CCA ATC GAG CT 3' | *(F)* S. *flexneri* 2b *min*CDE cloning |
| ENOL060 | *5' **(Tail)*** CTC CTG GC **(*Hind*III)** AAG CTT **(*min*E*,* includes last 2bp from *Hind*III)** ATT TCA GCT CTT CTG CTT CCG 3' | (*R*) *S*. *flexneri* 2b minCDE cloning |
| ENOL062 | *5' **(Tail)*** CTC TCA TAA **(*Sma*I)** CCC GGG **(*Xba*I)** TCT AGA **(*min*D)** GGC GTG ATC CCA GAG GAT CAA T 3' | *(F) S. flexneri* 2b deletion of minCDE to get Δ*min*CDE |
| ENOL063 | 5' **(*Tail*)** CTC TCA TTC **(*Sma*I-*Kpn*I)** CCC GGG TAC C ***(min*C*)*** TGT GGA GCA TAA ATA CGC TGA CC '3 | *(R) S*. *flexneri* 2b deletion of minCDE to get Δ*min*CDE |
| ENOL038 | *5' **(Tail)*** CTC CAG TCT **(*Hind*III)** AAG CTT **(*min*D)** AGG AGC CGC GCT TAC TAT TAG C 3' | *(R) Listeria monocytogenes* minCD |
| ENOL048 | *5' **(Tail)*** CTC CAG TCT **(*Sac*I)** GAG CTC ***(min*C*)*** GAA GAA GAA TGT TCA AAT TAA AGG C 3' | *(F) Listeria monocytogenes* minCD |
| ENOL039 | 5' ***(Tail)*** CTC CAG TCT **(*Bam*HI)** GGA TCC **(*Xba*I)** TCT AGA ***(min*C*)*** ATC CCC TGG AAC CTG AAC AAC 3' | *(R) Listeria monocytogenes* deletion of minCD to get *Δmin*CD |
| ENOL040 | 5' ***(Tail)*** CTC CAG TCT **(*Bam*HI)** GGA TCC **(*Kpn*I)** GGT ACC **(*min*D)** CCG GAA ATA TCA GCA GTT CG 3' | *(F) Listeria monocytogenes* deletion of minCD to get Δ*min*CD |
| ENOL098 | 5' ***(Tail)*** CTC CAG TCT ***(Xba*I*)*** TCT AGA **(*Cm*^{R})** TTT TTG CGC TTA AAA CCA GTC AT 3' | (R) obtain Cm' from pMK4 |
| ENOL099 | *5' **(Tail)*** CTC CAG TCT **(*Kpn*I)** GGT ACC **(*Cm*^{R})** AAA ACC TTC TTC AAC TAA CGG GG 3' | (F) obtain Cm' from pMK4 |
| ENOL096 | 5' ***(Tail)*** CTC CAG TCT ***(XbaI)*** TCT AGA ***(Em^{r})*** GAG ATA AGA CGG TTC GTG TTC GT 3' | (R) obtain Em^{r} from pVA838 |
| ENOL097 | 5' **(*Tail*)** CTC CAG TCT **(*Kpn*I)** GGT ACC **(*Em*^{r})** AGA ATG CAG AAG ATG AAA GCT GG 3' | (*F*) obtain Em' from pVA838 |
| ENOL092 | *5' **(Tail)*** CTC CAG TCT ***(Eco*RI*)*** GAA TTC **(*Kan*^{r})** TGA AGG ATG CTT AGG AAG ACG AG 3' | (*F*) obtain Kan' from pRB373 |
| ENOL093 | *5' **(Tail)*** CTC CAG TCT **(*Eco*RI)** GAA TTC ***(Kan*^{r}*)*** CGC CAT GAC AGC CAT GAT AA 3' | (R) obtain Kan' from pRB373 |
| ENOL094 | *5' **(Tail)*** CTC CAG CTC ***(Eco*RI*)*** GAA TTC **(*Kan*^{r} and G+ *ori*)** AAG GTG CGT TGA AGT GTT GGT AT 3' | (*F*) obtain Kan' and G+ve *ori* from pRB373 |

A deletion in the *min*CDE gene cluster was obtained using PCR primers ENOL062 and ENOL063 (Table 3) and plasmid pEN055 as the template DNA in a reverse PCR reaction. This resulted in the deletion of 271 bp (3' terminal region of *min*C)*,* 23 bp (inter-geneic sequence between *min*C and *min*D), and 608 bp (5' terminal region of *min*D). Simultaneously, the PCR primer sequences inserted a multiple cloning site carrying *Kpn*I*-Sma*I*-Xba*I restriction sites. The PCR product was digested with *SmaI* and religated to create plasmid pEN056.

The Cml^{R} marker was gel-purified from plasmid pHSG415 (Table 2) as a 1330 bp *Hae*II fragment, blunt-ended with T4 DNA polymerase and cloned into the *SmaI* site of plasmid pEN056. The new Δ*min*CDE::*Cml*^{R} plasmid was designated pEN057.

The Δ*min*CDE:*:Cml*^{R} cassette was obtained by PCR using primers ENOL059 and ENOL060 (Table 3) and using plasmid pEN057 as the template. The 2,272 bp fragment was blunt-end cloned into the EcoRV site of suicide plasmid pGP704 (Table 2). The new suicide plasmid was designated pEN060 and the SM10λpir strain carrying it was designated ENE105.

A filter-mating conjugation was used to transfer plasmid pEN060 from ENE105 to *Shigella flexneri* strain ENSf001 (Table 1) with a recipient:donor ratio of 5:1, overnight cultures (static growth) of the recipient and donor were mixed on Hybond N⁺ membranes on TSA plates at the calculated ratio and incubated overnight at 37°C. The cells were recovered and washed twice prior to plating out on selective minimal media containing supplements and 30 µg/ml chloramphenicol. The donor and recipient cannot grow on this media due to either auxotrophic requirements (donor) or antibiotic sensitivity (recipient) so that any colonies found should be exconjugants. Thirty-two (32) isolates were picked and patched onto fresh minimal media plates containing supplements and 30 µg/ml chloramphenicol.

The 32 exconjugants were incubated at 37°C overnight before being patched onto fresh minimal media plates containing supplements and 100 µg/ml ampicillin and then incubated at 37°C for 24-48 hours. All 32 isolates were able to grow on ampicillin indicating integration of the whole plasmid. Each isolate was examined under darkfield microscopy (40X) or under oil immersion (100X). Three of the isolates revealed the presence of minicells. The isolates were streaked onto XLD plates containing 30 µg/ml chloramphenicol to confirm that the isolate was *Shigella* and not the *E. coli* donor. The isolates also showed a strong positive agglutination reaction when tested with *Shigella flexneri* agglutinating sera (BIODESIGN International, Saco, Maine, USA). Plasmid purification and gel-electrophoretic analysis confirmed that the donor plasmid was not present in the exconjugants as an episome.

### [C] Generation of minicell producing strain from Escherichia coli.

Given that there is a 98 % genetic homology between *E*. *coli* and *Shigella* genomes this study aimed to determine if heterologous Δ*min*CDE gene sequences can be used to generate minicell producing strains especially where genome sequences are closely related. Therefore a filter-mating conjugation was used to transfer plasmid pEN060 (carries Δ*min*CDE::*Cml*^{R} from *S*. *flexneri* 2a; Fig. 3) from ENE105 (Table 1) to *E*. *coli* strain ENIh003 (JM109; Table 1) with a recipient:donor ratio of 3:1. Overnight cultures (static growth) of the recipient and donor were mixed on Hybond N⁺ membranes on TSA plates at the calculated ratio and incubated overnight at 37°C. The cells were recovered and washed twice prior to plating out on selective minimal media containing supplements and 30 µg/ml chloramphenicol. The donor and recipient cannot grow on this media due to either auxotrophic requirements (donor) or antibiotic sensitivity (recipient). 106 isolates were picked and patched onto fresh minimal media plates containing supplements and 30 µg/ml chloramphenicol. After overnight incubation at 37°C the isolates were patched onto fresh minimal media plates containing supplements and 100 µg/ml ampicillin and incubated at 37°C for 24-48 hours. All 106 isolates were able to grow on ampicillin suggesting integration of the whole plasmid. Twenty-four (24) isolates were viewed under darkfield microscopy (40X) and 100X oil immersion. Six isolates showed the presence of large numbers of minicells. Although many different general mutagenesis and site-directed mutagenesis protocols have been employed in the past to generate minicell producing bacterial strains, this invention is the first to demonstrate that the unique cloning/site-directed mutagenesis protocol employed in this study is versatile and can be reliably used to generate minicell producing strains from a range of Gram-negative and Gram-positive bacteria (example 2 shown below).

### Example 2. Generation of minicells from Listeria monocytogenes

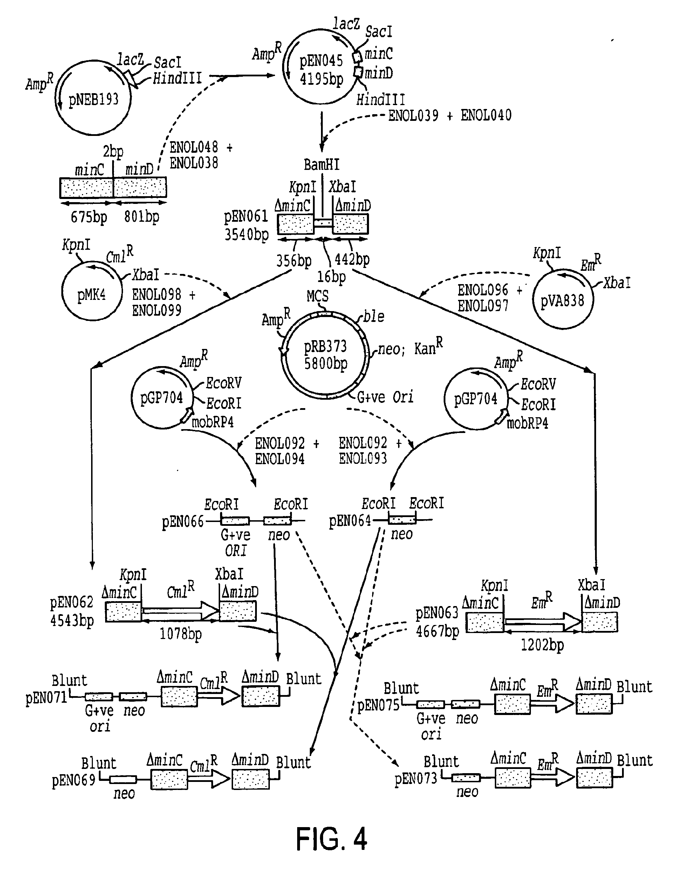

Minicell-producing bacterial strains from Gram-positive bacteria can be generated as described in this example. A schematic diagram of plasmid construction is shown in Figure 4. The bacterial strains, plasmids and PCR primers are respectively listed in Table 1, Table 2, and Table 3.

To clone the *min*CD genes from the genome of *L. monocytogenes,* PCR was performed using primers ENOL038 and ENOL048 (Table 3) and purified *L*. *monocytogenes* genomic DNA as template. PCR reactions were carried out in 50 µl volumes using the Platinum^{®} P*fx* DNA Polymerase kit (Invitrogen Corporation, Carlsbad, CA, USA). Reactions included 1X P*fx* buffer, 2 mM MgSO₄, 0.2 mM dATP, dTTP, dGTP and dCTP, 50 pmol of each primer and 1U of P*fx* polymerase. Cycling conditions included a 94°C denaturing step for two minutes; followed by 35 cycles of 94°C for 30 seconds, 55°C for 30 seconds and 68°C for two minutes; followed by a 68°C final extension step for five minutes. A 2-5 µl sample of each PCR reaction mixture was visualised on a 1 % agarose gel stained with ethidium bromide. The amplified *min*CD fragment was purified by excision from the agarose gel, followed by electro-elution using the Model 422 Electro-Eluter (Bio-Rad Laboratories, Hercules, CA, USA) according to the manufacturer's specifications.

The *minCD* fragment (1,556 bp) was digested with *Sac*I and *H*i*nd*III, and directionally cloned into the respective sites of plasmid pNEB193 (Table 2; Fig. 4). Recombinant clones transformed in strain ENIh003 (Table 1) were characterized by restriction digestion and gel-electrophoretic analysis. The correct clone (plasmid designated pEN045) was sequenced to confirm the identity of *minCD* genes.

PCR deletion was performed on the cloned *minCD* fragment in plasmid pEN045 using primers ENOL039 and ENOL040 (Table 3). The primers carry restriction sites *XbaI* and *KpnI* respectively, which serve as insertion sites for selection markers between Δ*min*C and Δ*min*D. Reaction conditions for deletion of *min*CD, and fragment visualization, were as described above, but with a 4 minute 68°C extension step. The Δ*min*CD fragment was subsequently digested with *Xba*I and *Kpn*I*,* and purified by electro-elution with the Model 422 Electro-Eluter as preparation for ligation with selection markers.

Two different antibiotic selection markers from Gram-positive bacteria were inserted between the Δ*min*C and Δ*min*D genes in pEN045 at the *Kpn*I and *Xba*I sites. The chloramphenicol resistance marker (Cml^{R}) from the *Bacillus subtilis* plasmid pMK4 (Table 2), and the erythromycin resistance marker (Em^{R}) from the *Streptomyces sanguis* plasmid pVA838 (Table 2) were used to construct plasmids pEN062 and pEN063 respectively (Fig. 4). Each marker was amplified by PCR using the oligonucleotides listed in Table 2 and shown in Fig. 4, which include *Xba*I and *KpnI* sites. PCR of Cm^{R} and Em^{R} was carried out using the same reaction volume and reagent concentrations as described above. Cycling conditions included a 94°C denaturation step for two minutes; followed by 35 cycles of 94°C for 30 seconds, 55°C for one minute and 68°C for two minutes; followed by a 68°C extension step for five minutes. PCR fragments were gel purified using the MinElute kit (Qiagen Inc, Victoria, Australia) according to the manufacturer's instructions. Cm^{R} and Em^{R} were then digested with *Kpn*I and *Xba*I*,* purified with a MinElute column and ligated to the respective sites between the Δ*min*C and Δ*min*D genes in pEN045. JM109 recombinants were characterized by restriction digestion with the enzymes *Eco*RI and *Dde*I (Cm^{R}) and *Eco*RI and *Ava*I (Em^{R}) to give strain isolates ENE110 and ENE112 respectively.

For the purpose of conjugation experiments between *L. monocytogenes* and *E. coli* SM10λ pir (ENIh001), four new plasmids were constructed, using the conjugative plasmid pGP704. The plasmid pGP704 was modified to include either the kanamycin resistance marker (Km^{R}), encoded by the *neo* gene, or, Km^{R} as well as the *ori* for plasmid replication in Gram-positive bacteria. The former would act as a suicide plasmid in the absence of a Gram-positive origin of replication, with the *neo* gene (Km^{R}) serving as a marker for plasmid presence. In contrast, the latter would act as a positive control for conjugation experiments.

DNA segments containing the *neo* gene and the *neo* gene including Gram-positive *ori* were amplified by PCR from pRB373 using the set of oligonucleotides listed in Table 3. PCR reaction volumes and reagent concentrations were as described above. Cycling conditions included a denaturation step of 2 minutes at 94°C; followed by 35 cycles of 94°C for 30 seconds, 56°C for one minute, and 68°C for 2 and a half minutes; followed by a long extension step of 68°C for 5 minutes. Products were purified from an agarose gel using the MinElute column. The fragments containing the *neo* gene and *neo* gene plus the Gram-positive *ori* were cut with *Eco*RI and ligated into the *Eco*RI site of pGP704 (Fig. 4). This resulted in plasmids designated pEN064 and pENO66.

The Δ*min*CD::*Cml*^{R} or Δ*min*CD::*Em*^{R} were then excised as *Sac*I *- Hind*III fragments from pEN062 and pEN063 respectively, blunt ended and ligated into the *Eco*RV site of plasmids pEN064 and pEN066, to give the four different plasmids pEN069, pEN071, pEN073 and pEN075 as depicted in Fig. 4. The four plasmids were transformed into ENIh001 (*E. coli* SM10λ pir) to create the strains ENE124, ENE126, ENE128 and ENE130 respectively. Thus ENE124 contained the plasmid with the Δ*min*CD::Cm^{R} and the *neo* gene (Km^{R}), while ENE126 has, in addition, the Gram-positive *ori.* Similarly, ENE128 contained the plasmid with the Δ*min*CD::Em^{R} and the *neo* gene (Km^{R}), while ENE130 additionally contained the Gram-positive *ori.*

The four strains ENE124, ENE126, ENE128 and ENE130 were used as donor strains for conjugation experiments with *L. monocytogenes* (ENLM001). Conjugations were carried out essentially as described in Trieu-Cuot *et al.* (1991). All strains were grown to mid-log phase and mixed in a 2:1 ratio (recipient:donor) to a 1 ml volume. Conjugation mixes were washed twice in BHI before resuspending in 1 ml BHI. 200 µl volumes were plated on 0.45 µM nitrocellulose membrane filters (Millipore) on BHI plates and incubated for 18 hours at 37°C. Following incubation, the nitrocellulose membranes were sliced into strips and placed in 3 ml BHI. Vigorous vortexing was applied to dislodge bacterial cells from the filter membranes. Samples of 300 µl volume were plated out on large plates containing BHI, Nalidixic acid (Nal; 50 µg/ml), colistin (Col; Polymixin E) (10 µg/ml) and either chloramphenicol (10 µg/ml) or erythromycin (10 µg/ml). Plates were incubated for 48 hours at 37°C before picking of colonies.

Colonies were patched onto BHI/Nal/Col plates that contained kanamycin (15 µg/ml), as well as onto plates with BHI/Nal/Col and either chloramphenicol (10 µg/ml) or erythromycin (10 µg/ml), for antibiotc sensitivity testing. All ex-conjugants demonstrated an antibiotic profile that suggested the chromosomal integration of the *min*CD deletion without integration of the plasmid. That is, all ex-conjugants grew on antibiotic plates containing the internal marker erythromycin or chloramphenicol and no ex-conjugants grew on plates containing kanamycin (Km^{R} encoded by the *neo* gene on donor plasmids).

Over one hundred ex-conjugants were examined for the minicell phenotype, using dark field microscopy (40x) and oil immersion (100x). All isolates demonstrated a varying number of minicell structures among the population of *L*. *monocytogenes* parent rods, when compared to parent cells under the microscope (ENIh001 and ENLm001). This result also is consistent with integration of the *min*CD deletion and disruption of normal pericentral division.

Thirty ex-conjugants were chosen and subcultured to test for maintenance of the minicell phenotype. Upon confirmation of maintenance, the isolates were stored as glycerol stocks for future experiments.

### Example 3. Purification of minicells from bacterial species

Minicells were purified by the following inventive method. This example details purification of *S*. *typhimurium min*CDE*-* derived minicells. The same procedure was used to purify minicells from additional *min* mutant strains, including two mutants of *S*. *typhimurium,* and one mutant each of *E*. *coli, S. flexneri* and *L. monocytogenes.* The process was optimized and repeated more than 50 times to generate purified minicells. It was reliable, and routinely yielded 10⁸ to 10⁹ purified minicells from a 10 L bacterial culture.

A *S*. *typhimurium min*CDE-/pEGFP-C1 culture was established from a glycerol stock in 50 ml TSB containing antibiotics Chloramphenicol and Kanamycin (50 ug/ml final concentration). The culture was incubated with shaking at 37°C overnight. A 2.5 ml aliquot of the overnight culture was used to inoculate 1L (in a 2L baffled conical flask) of TSB containing the above-mentioned antibiotics, and five flasks were incubated with shaking at 37°C overnight.

### (A) Pre-Preparation (Stage I)

A 100 L Bioprocess bag was filled with Type 1 water (MQ) by sterile hose via a 0.2 µm filter. Into two 20-liter carboys, previously autoclaved, that contained 2 L of 10X BSG, 18 L of sterile process water was transferred by peristaltic pump. One carboy was for diluting the minicell suspension and the other was for use in diafiltration.

### (B) Differential Centrifugation and Pre-Preparation (Stage 2)

The bacterial culture was centrifuged at 2000g for 10 minutes (Sorvall Legend T/RT; TTH 750 rotor). The supernatant was decanted into a sterile 5 L carboy that was fitted with a 0.2 µm breather filter and a quick disconnect fitting. The decantation procedure was performed in a Class II biohazard cabinet. The carboy was sealed, sterile tubing was connected to the 5 L carboy, and the other end of the tubing was connected to the pre-filled 20 L carboy containing 20 L of 1 x BSG as described above. The minicell-carrying suspension was pumped from the 5 L carboy into the 20 L carboy, to give a dilution of 1:5.

### (C) Continuous Minicell Purification System

Three cross-flow systems from Sartorius were connected in series. In duplicate, 0.45 µm Sartocon Slice Cassette filters were fitted in the first two slice holders, and a 0.2 µm Sartocon Slice filter cassette was fitted in the last holder. The torsion on each filter unit was tightened to 20 Nm (Newton meters), using a torsion wrench. Each unit was attached to a pump via a sanitary element. Feed, retentate and permeate lines were connected. Prior to attachment of carboys, the entire system was internally washed with 6 L of 1N NaOH at 2 bar pressure for 15 minutes. This step internally sterilized the various hoses and filters. The system was drained of NaOH by reversing the pump direction of liquid flow and a water flux-rate test was performed to ensure proper filter cleaning. The test was performed according to the manufacturer's instructions (Sartorius manual). Acceptable flux-rates of 3,000 to 3,600 ml per minute for the retentate and 600 to 800 ml per minute for permeate were ensured prior to performing the minicell filtration. The system still carried a high pH and hence this was neutralized by flushing and recirculating through each system with sterile 1 x PBS (pH 7.4) until the measured pH of the PBS pool was in the range of 7.0 to 8.0. Carboys (20 L) were then connected. The minicell suspension (25 L) was carried in a first carboy, which was connected via a hose to the first 0.45 µm filter cassette. To prevent filter fouling, the minicell suspension was diluted as the filtration step proceeded (i.e., diafiltration). The diluent (20L of 1 x BSG) was carried in a second carboy. Therefore, in the first cross-flow filtration step, the minicell suspension was filtered through in a volume of 45 L. The permeate valve was initially closed and the minicell suspension was pumped over the surface of the 0.45 µm filter at 2 bar pressure for 5 minutes. This conditioned the filter for the minicell suspension medium. The permeate valve was then opened and the minicell suspension was permeated (2 bar pressure, 600 ml per min) through the 0.45 µm filter and the permeate was collected in a third carboy. As the volume in the first carboy decreased, the amount of non-filtered solids increased, and hence diafiltration was switched on when the volume in the first carboy dropped to 15 L. This diluted the solids in the first carboy, preventing filter fouling and maximizing minicell recovery in the third carboy. Once the volume of permeate in the third carboy reached approximately 12.5 L, the second 0.45 µm cross-flow filter was conditioned for the minicell suspension found in the third carboy. When the volume in the third carboy reached the 15 L mark, the permeate valve was opened, allowing permeation of the minicell suspension into a fourth carboy.

At this stage, the larger parent bacterial cell contamination in the minicell suspension was removed. The next stage was to eliminate smaller contaminants in the suspension such as bacterial blebs, free endotoxin, nucleic acids, cellular debris, and excess liquid. This was accomplished via filtration through a 0.2 µm cross-flow filter. Minicells are approximately 0.4 µm in diameter and, hence, do not permeate through a 0.2 µm pore size. Bacterial blebs, on the other hand, range in size (diameter) from 0.05 µm to 0.2 µm and hence are filtered out. Other contaminants also are less than 0.2 µm and hence the only constituents retained in this filtration step were the minicells and any residual parent bacterial cells.

When the volume in the fourth carboy reached approximately 15 L, the 0.2 µm cross-flow filter was conditioned for the minicell suspension present in the fourth carboy. The permeate valve then was opened, allowing permeate to go to waste in a fifth carboy while the minicells were retained and concentrated in the fourth carboy. Due to incorporation of the diafiltration system, the minicells were continually being diluted and filtered, which ensured complete removal of contaminants at the end of the process. The concentration step therefore reduced the minicell suspension volume to approximately 4 L from the starting volume of 45 L.

### (D) Buffer Exchange for the Minicell Suspension

The residual salts, media components and low molecular weight wastes in the minicell suspension were eliminated by diafiltration with 1 x BSG. The apparatus was assembled and equilibrated as described before. The minicell suspension was placed in a first 4 L carboy, and 20 L of sterile 1 x BSG (diafiltration medium) were placed in a second carboy. The cross-flow unit was assembled with two 0.1 µm filter cassettes to ensure that the minicells were unable to pass through but all contaminants less than 0.1 µm were eliminated. The pump was switched on and speed adjusted to provide 0.5 bar pressure. The permeate valve was opened, and the minicell suspension flowed through the feed line, over the 0.1 µm filter. Minicells returned to the first carboy via the retentate line. The waste flowed through the permeate line and was collected in a third carboy. This reduced the volume of the minicell suspension and hence the diafiltration system was switched on to pump 1 x BSG into the first carboy. This step continuously replenished the volume of the minicell suspension to keep it at 4 L. The procedure was continued until the second carboy was emptied, resulting in five changes of the minicell suspension buffer.

### (E) Sterilizing Filtration of the Minicell Suspension

At this stage, the minicell suspension still carried some parent bacterial contamination because the 0.45 µm cross-flow filters were not sterilizing filters. Therefore, it was important to eliminate any residual parent bacteria to obtain a minicell suspension that was optimal for *in-vitro* and *in-vivo* use. The 4 L minicell suspension from the previous step was initially diluted to 20 L in sterile 1 x BSG and was held in a first carboy. A dead-end filter unit carrying a 0.45 µm filter with a large surface area (500 cm²) was pre-wetted with 2 L of sterile 1 x BSG and integrity tested according to the manufacturer's instructions. The minicell suspension was pumped at a flow rate of 700 ml/min (i.e., slow flow rate to prevent forcing parent bacterial cells through the 0.45 µm filter) through the dead-end filter. Bacterial cells were retained by the filter, whilst the minicells flowed through into a second carboy via the filtrate line.

### (F) Concentration of Purified Minicells

The purified minicells, in a 20 L suspension, were concentrated to a smaller volume. This step was not readily accomplished by standard centrifugation and pellet resuspension technique, however, because the volumes were large and, in practice, not conducive to centrifugation technique.

The concentration step was performed in the following four stages.

**Stage 1:** The minicell suspension was pumped at 0.5 bar pressure out of a first carboy and over a 100 kDa cross-flow filter via a sanitary element. The minicells were returned to the first carboy via a retentate line, and the liquid permeate was collected in a second carboy via a permeate waste line.

**Stage 2:** Once the minicell suspension volume was reduced to 4 L, the process was stopped and the suspension was transferred into a third sterile 4 L carboy. The latter was fitted with 6.4 mm diameter hoses compared to the 12.5 mm hoses used for handling the larger volumes above. This reduced the void volume of the tubing. Because the feed and retentate lines were 12.5 mm diameter tubing, an adapter was designed to fit the hose in lid closure. Similarly, an adapter was designed to fit the larger bore tubing of feed and retentate lines. This second concentration stage was performed as in the previous stage until the minicell volume was further reduced to approximately 200 ml.

**Stage 3:** The 200 ml minicell suspension was transferred into a modified Schott bottle that contained sterile internal glass tubing for feed and retentate. The glass tubing was bored through the cap of the Schott bottle, and was sealed with Marprene tubing and silicon. On the cap, the bottle also carried a breather filter (0.2 µm). To reduce void volume further, the previously used feed, sanitary element and retentate tubing was replaced with sterile 6.4 mm Marprene tubes, and the previously used pump was replaced with a smaller one. The process of concentration was performed as before (100 kDA cross-flow filter) until the minicell volume was reduced to 50 ml.

**Stage 4:** The highly purified minicell suspension was transferred under sterile conditions into a 50 ml Falcon tube.

### Example 4. Scanning electron microscopy characterization of minicells from Gram-negative and Gram-positive minCD- strains

Scanning (SEM) and Transmission (TEM) electron micrographs of minicells derived from *S*. *typhimurium, E. coli, S. flexnieri, L. monocytogenes and the corresponding* parent cells were taken to determine the morphology and dimensions of the various minicells. Briefly, bacterial cultures carrying minicells were centrifuged at 13,200 rpm for 20 minutes and resuspended in PBS containing 2.5% glutaraldehyde and fixed at room temperature for 40 minutes. Samples were centrifuged and washed three times in distilled water. For TEM, the following procedure was followed. To change solutions the cells were centrifuged at 10,000 rpm for 1 minute, the supernatant was pipetted off, then the cells were resuspended in the new reagent using a vortex mixer. The sequence of reagents was: (a) osmium tetroxide in 0.1 M cacodylate buffer at pH 7.2 - 10 minutes, (b) sodium acetate 4% in distilled water - 1 minute, (c) uranyl acetate 4% in distilled water - 5 minutes, (d) 70% ethanol - 5 minutes, (e) 100% ethanol - 5 minutes, (f) 100% acetone - 5 minutes, (g) 1:1 acetone and Spurr's epoxy resin monomer - 30 minutes, (h) pure Spurr's epoxy resin - cure for 48 hours at 60°C. Sections were cut from the cured resin blocks with a diamond knife using a Reichert Ultracut E ultramicrotome. Sections were stained with uranyl acetate for 10 minutes followed by lead citrate for 2 minutes. The sections were examined using a Hitachi H-7000 transmission electron microscope operated with a beam energy of 75 kilovolts (University of New South Wales, NSW, Australia). Digital images were recorded using an AnalySis MegaView II widefield CCD camera.

For High Resolution Scanning Electron Microscopy the following method was followed. To change solutions the cells were centrifuged at 13,000 rpm for 20 minutes, the supernatant was pipetted off, and the cells were resuspended in the new reagent using a vortex mixer. The intention was to wash all ions and biomaterials off the cells and leave them suspended in a small volume of distilled water. The sequence of reagents was (a) 1 ml of distilled water - repellet, (b) 1 ml of distilled water - resuspend, (c) deposit 250 µl on a clean brass specimen plate, (d) dry overnight at 30°C, (e) coat just before microscopy with 2 nm of chromium metal deposited in a Xenosput clean vacuum sputter coater. The coated specimens were examined using an Hitachi S-900 Field Emission Scanning Electron microscope using a beam energy of 3 kilovolts (University of New South Wales, NSW, Australia). Digital images at different magnifications were recorded using an ImageSlave digitiser.

The results showed that the minicells derived from both the Gram-negative and Gram-positive bacteria were about 400 nm in diameter, and except for *L*. *monocytogenes* they appeared to have a ruffled surface as seen by SEM, presumably due to the lipopolysaccharide cell surface structures. There was no apparent difference in the surface ultrastructure of minicells and the parent bacteria for all species. TEM results showed that *L. monocytogenes* minicells had a rigid cell wall structure expected of a Gram-positive bacterial cell membrane. *Salmonella, S. flexneri* and *E*. *coli* membranes collapsed more readily under the microscope electron beam. The minicell formation event, *i.e*., asymmetric cell division, was observed in all samples.

### Example 5. Minicell uptake by mammalian cells such as macrophages

To demonstrate the uptake of recombinant minicells by macrophages, it was first necessary to incorporate a tracer such as GFP, in order that the recombinant minicells could be seen, distinct from the mammalian cells. Therefore, plasmid pEGFP was transformed into the *S*. *typhimurium, E. coli* and *S*. *flexneri min*CDE-minicell producing strains to determine if minicells would stably carry EGFP and fluoresce green.

Plasmid pEGFP (Table 2) is a bacterial expression plasmid that expresses a red-shifted variant of wild-type green fluorescent protein (EGFP; excitation maximum: 488 nm; emission maximum: 507 nm) from the *lac* promoter. The plasmid backbone is the pUC19 derivative, pPD16.43 (Fire *et al.,* 1990), which provides a high-copy-number origin of replication and an ampicillin resistance gene for propagation and selection in bacterial cells. The plasmid was transformed into *S. typhimurium, E. coli* and *S. flexneri min*CDE⁻ strains and recombinant bacteria were grown in Brain Heart Infusion broth (BHI; Difco Laboratories, Detroit, Michigan USA) until an OD₆₀₀ of 0.6 was reached. The minicells were isolated and purified from the mixed culture and were visualized by fluorescent microscopy (Fluorescence microscope DMLM, Leica Microsystems). The results revealed that all minicells fluoresced bright green, while minicells purified from non-recombinant *S. typhimurium, E. coli* and *S*. *flexneri min*CDE⁻ strains (controls) did not show any green fluorescence. The stored minicells (4°C and room temperature) were viewed by fluorescence microscopy at time intervals of 30 minutes, 1 hour, 6 hours, 12 hours, 18 hours, 24 hours, 2 days, 1 week, and 2 weeks. The results showed that the minicells were intact and continued to fluoresce bright green throughout the study. The plasmid pEGFP therefore provided a tracer (minicells fluorescing green) with which to follow the uptake of recombinant minicells by mammalian cells, such as macrophages, and other cells such as cancer cells. These results indicate that recombinant proteins, once expressed or segregated into minicells, are stable for a significant period of time (probably until minicell cellular integrity is compromised) due to the absence of chromosomally encoded proteases.

Cells of the mouse macrophage cell line RAW264.7 (Ralph & Nakoinz, 1977) obtained from the American Type Culture Collection (ATCC), were cultured *in vitro* at a cell density of 10⁵ per well, and were infected with purified recombinant minicells, carrying plasmid pEGFP, that were derived from *S*. *typhimurium, E. coli,* and *S. flexneri,* at a minicell:macrophage ratio of 50:1 and 100:1.

Prior to macrophage infection, the purified minicells were visualized via fluorescence microscope to confirm that most minicells carried EGFP and hence fluoresced bright green. As a positive control, *S*. *typhimurium aro*A- strain SL3261 (Table 1) carrying the same plasmid was also used to transfect the macrophage cells at the same bacteria:macrophage ratio. Negative control (uninfected macrophages) were also processed in the same way as experimental infected cells. The culture plates were centrifuged at 1000g for 10 minutes at 37°C. For the minicell transfection study, the antibiotics gentamycin (100 µg/ml) and ampicillin (200 µg/ml) were added to kill any residual live parent bacterial cells. The positive control salmonellae were also killed with the same antibiotic treatment. The plates were incubated at 37°C for 30 minutes in 5% CO₂, followed by three washes in PBS. For the *S. typhimurium* derived minicell/macrophage experiment, the slides were fixed with 4% formaldehyde for 40 minutes and then permeabilized with 0.2 % Triton X-100. After blocking non-specific staining with 5% normal goat serum (NGS) in phosphate buffer containing 5% BSA, the coverslips were incubated with anti-lipopolysaccharide (LPS) antibody (rabbit 4-O *Salmonella* somatic agglutinating serum; 1:200 dilution; Murex Biotech, Dartford, England) for four hours at room temperature. The cells were washed three times in PBS and incubated with secondary antibody (1:1000), Alexa Fluor 594 (Molecular Probes, Eugene, OR, USA; goat anti-mouse IgG conjugate, excitation: 590 nm, emission: 617 nm) in PBS-BSA. Plates were incubated with second antibody for 1 hour in the dark and were washed three times with PBS for 5 minutes each. Coverslips were mounted with glycerol and viewed by confocal microscopy.

The results showed that *S*. *typhimurium, E. coli* and *S*. *flexneri*-derived minicells were all engulfed by approximately 20% to 30% of the macrophage cells in culture. The minicells fluoresced bright green and were associated with the macrophages. The control non-recombinant *S*. *typhimurium, E. coli and S. flexneri-*derived minicells did not reveal green fluorescent dots associated with macrophages other than minor non-specific background fluorescence. Control recombinant *S*. *typhimurium aro*A*-* strain also gave a similar result to that seen with both the recombinant minicells.

To confirm that the green fluorescent dots were within the macrophage cells, *i.e.,* were engulfed minicells, and not just adhered to the cell surface, three-dimensional images (using sagittal and coronal sections) were taken for the S. *typhimurium*-derived minicell-macrophage interaction. In both coronal and sagittal sections, the minicells were localized within the macrophages, indicating that the minicells had been engulfed by the macrophages. Additionally, anti-04 LPS labeling of the green fluorescent dots (yellow fluorescence following secondary antibody Alexa Fluor 594) showed that the green fluorescent dots in fact were EGFP-expressing minicells and not artifact background fluorescence. A similar result was observed with the positive control salmonellae, demonstrating that the bacterial surface structures required for receptor-mediated uptake of the cells by macrophages were conserved on the minicell surface.

### Examples 6. Minicell uptake and breakdown in macrophage phagolysosomes

To demonstrate the intracellular fate of minicells within macrophages, TEM studies were carried out on mouse macrophages infected with *S*. *typhimurium-*derived minicells.

Briefly, mouse macrophage cell line RAW 246.7 was grown to 50% confluence in T25 flasks in standard culture media. Minicells on the order of 10⁷, in 100 µl, were added directly to media in flasks, and the process of macrophage infection was carried out as described in Example 5. An approximate ratio of minicell:macrophage of 10:1 was used. Cells were collected for time points corresponding to 30 minutes, 60 minutes, and 2 hours post-infection. An additional flask was also included as a negative control, receiving no minicells. Cells were trypsinised & pellets collected and fixed in 4% glutaraldehyde (500 µl). Samples were processed and analyzed by TEM (University of New South Wales, Sydney, Australia).

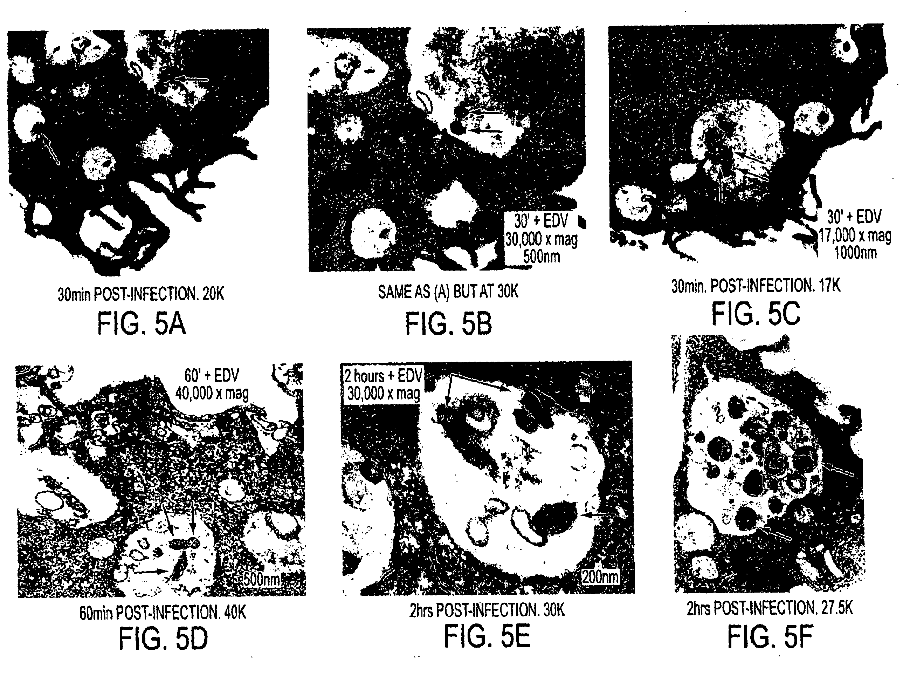

The results showed that as early as 30 minutes post-infection, electron-dense particles approximately the size of minicells (400 nm) were observed within macrophage vacuoles (Figure 5, panels A-F). With the progression of time (60 minutes and 2 hours), the electron-dense particles appeared less intact with surface irregularity and loss of electron-density.

To confirm that the intra-vacuolar electron-dense particles were engulfed minicells, the above experiment was repeated with a difference being that after the various time intervals post-infection, cells were fixed (4 % paraformaldehyde, 0.1 % glutaraldehyde) for 30 minutes at room temperature, washed with PBS and collected into 1.5 ml PBS by gentle cell scraping. Samples were processed for immunogold-TEM (EM Unit, ICPMR, Westmead Hospital, Sydney, Australia). Samples were gently pelleted and processed by freeze-substitution method. Briefly, the samples were labeled with primary antibody (anti-*S*. *typhimurium* lipopolysaccharide [Factor 4, Group B specificity]; Abbott Murex, USA) 1:200 dilution, followed by gold (10 nm) conjugated secondary antibody. The samples were viewed using a Philips CM-120 BioTWIN electron microscope at 80 kV. Images were captured onto type 4489 Kodak EM emulsion film.

The results showed that the minicells were clearly identified by the gold-labeled anti-O4-LPS antibody and that the electron-dense particles observed in the macrophage vacuoles were the *S*. *typhimurium*-derived minicells. No gold-labeling was observed in control macrophages that had not been infected with minicells. This data also revealed that at later time points, gold particles not associated with minicells were observed in the vacuoles. There was a marked increase in the minicell-free gold particles at later time-points and this was also associated with increased numbers of minicells that had lost cell wall integrity and cellular electron-density. These data indicate that the minicells follow the classical pathway of antigen-uptake and processing exhibited by macrophages, which includes foreign particle ingestion into early endosomes followed by endosome-lysosome fusion and breakdown of the antigen in the acidic phagolysosome. The minicell-free gold particles in the late stages of infection may indicate LPS that is released from processed or digested minicells.

These results show that recombinant minicells not only are engulfed by mammalian cells, such as macrophages, but also are degraded in intracellular vacuoles, presumably phagolysosomes.

### Example 7. Expression of heterologous protein by minicell transfected macrophages

To determine if recombinant minicells carrying a mammalian gene expression plasmid encoding EGFP (not expressed in bacterial cells or minicells) could deliver the plasmid to the mammalian cell nucleus and achieve expression of EGFP in the mammalian cell, the following experiment was performed.

Cells of the mouse macrophage cell line RAW-264.7 were cultured *in vitro* and infected with purified, recombinant *S*. *typhimurium, E. coli* and *S*. *flexneri-*derived minicells carrying plasmid pEGFP-C1 (Table 2), as described in Example 5. Forty-eight hours post-transfection, the infected cells were visualized by three-dimensional confocal microscopy.

The results showed that approximately 20% of the macrophages fluoresced green, suggesting that the recombinant minicells were broken down within the macrophages, presumably in phagolysosomes, and that at least some of the released plasmid DNA was taken up by the cell nucleus prior to expression of the green fluorescent protein. Control macrophages, *i.e*., macrophages transfected with non-recombinant minicells, did not reveal the green fluorescence. EGFP expression in the experimental cells took at least 48 hours. This result was similar to that observed with positive control macrophages transfected with plasmid pEGFP-C1 using electroporation with a BioRad Genepulser.

To confirm further that EGFP expression within the minicell-transfected macrophages was not background fluorescence, this experiment was repeated for S. *typhimurium*-derived minicells. In this case, after fixation with formaldehyde, the cover slips were incubated with anti-GFP monoclonal antibody (Clontech Laboratories, Palo Alto, CA, USA; 1:300 dilution) and incubated overnight at 4°C. The cover slips were washed three times with PBS (5 minutes per wash) and incubated with 2 % normal goat serum in PBS/BSA for 20 minutes. The cover slips were washed twice with PBS and treated with secondary antibody Alexa Fluor 594-anti-mouse IgG conjugate in PBS (1:1000 dilution). The reaction was incubated in the dark for 1 hour and washed twice in PBS. The cover slips were visualized by confocal microscopy using red (570 nm) and green (488 nm) fluorescence visualization filters. The results revealed that that the spots of green fluorescence (laser excitation at 488 nm) observed within the macrophage were identical to the spots of red fluorescence (laser excitation at 570 nm). When both lasers were used, the green and red fluorescence signals were co-localized, and appeared as a yellow fluorescence. Additionally, when the 3D image was constructed using Leica 3D image software, the fluorescence was found to be within the macrophage. These results show that the observed green fluorescent spots were due to macrophage expression of EGFP protein since the same spots were identified by anti-GFP monoclonal antibody (red fluorescence).

The results confirm that recombinant minicells do break down in host mammalian cells such as macrophages, releasing plasmid DNA and that this DNA is able to express foreign protein within the mammalian cell. This demonstrates the feasibility of *in vitro* gene therapy by means of recombinant, intact minicells.

After recombinant minicells have been introduced into a patient, the presence of the heterologous gene product can be monitored or assessed by an appropriate assay for the gene product in the patient, for example in peripheral red blood cells of the patient when expression is erythroid cell-specific. As described above, the choice of assay is partly a function of the heterologous gene product and can be determined readily.

### Example 8. Minicell-mediated gene delivery to and gene expression in human breast cancer cells

Minicells purified from recombinant *S*. *typhimurium min*CDE- strain carrying plasmid pEGFP-C1 (eukaryotic gene expression only; Table 2) were used to infect human breast cancer cells (SK-BR-3). Forty-eight hours and 96 hours post-transfection, the cells were visualized via confocal microscopy. As a negative control, non-recombinant minicells were used to transfect SK-BR-3 cells and were visualized similarly to experimental cells.

SK-BR-3 breast cancer cells (source ATCC, reference No. HTB-30) were cultured on coverslips in 6-well plates and grown to approximately 50% confluency. Minicells carrying eukaryotic GFP-expression plasmid pEGFP-C1 were added to cells and centrifuged for 10 minutes, 1000g to allow minicell / SK-BR-3 cell contact. Cells were cultured for 48 hours after which G-418 (400 mg/ml) was added, with some wells receiving no G-418. After a further 48-hour incubation, all coverslips were fixed with 4% formaldehyde for 1 hour. The coverslips were incubated with PBS-BSA (2 % BSA in PBS) for 20 minutes and washed once with PBS. The coverslips were incubated overnight at 4°C with anti-Her-2 antibody (Serotec, monoclonal mouse anti- human IgG; 1:100 dilution). The cells were washed three times with PBS for 5 minutes each wash, and were incubated for 1 hour in the dark with Alexa Fluor 594-conjugated secondary antibody (Molecular Probe, goat anti-mouse IgG conjugate, excitation: 590 nm, emission: 617 nm; 1:1000 dilution in PBS-BSA). The cells were washed three times with PBS for 5 minutes per wash, and coverslips were treated with antifade medium (Molecular Probe). All coverslips were visualized by three-dimensional confocal microscopy (excitation with wavelengths for red filter: 568 nm and green filter: 488 nm).

The results revealed that approximately 10% of the breast cancer cells clearly expressed the Green Fluorescence Protein that was localized in the cytosol (see Figure 6, panels A-C). This was clearly visible over normal background autofluorescence exhibited by control cells. The cells were clearly identified with the anti-Her-2 antibody (red fluorescence).

This result demonstrates that recombinant minicells are able to deliver mammalian gene expression plasmid DNA to non-phagocyte cells, exemplified by epithelial breast cancer cells, in a manner that leads to heterologous expression within the cells.

### Example 9. Minicell-mediated gene delivery and gene expression in vivo in Balb/c mice

To determine that recombinant minicells could deliver a foreign gene to cells of the immune system *in vivo,* mice were vaccinated intraperitoneally with recombinant *S*. *typhimurium min*CDE- derived minicells carrying plasmid pEGFP-C1 (eukaryotic gene expression only; Table 2) and compared to mice vaccinated with *S*. *typhimurium* Δ*aro*A strain bearing the same plasmid.

Recombinant minicells were purified as in Example 3. *S*. *typhimurium* (SL3261 Δ*aro*A strain, Table 1) was prepared as follows. Five (5) ml Trypticase Soy Broth (TSB, Becton Dickinson, Palo Alto, CA, USA) was inoculated with a 1:100 inoculum of an overnight culture of *S*. *typhimurium* in TSB, and grown at 37°C with shaking until the Optical density (O.D.) measured at 600 nm reached 0.5. The bacteria were then incubated with gentamycin (Sigma-Aldrich, Castle Hill, NSW, Australia) at 150 µg/ml and ampicillin (Roche) at 150 µg/ml for a further 2 hours at 37°C with shaking.

The killed bacteria and minicells were pelleted by centrifugation at 8000 rpm, resuspended and washed a further three times in BSG (phosphate buffered saline [PBS; 1.44 gm disodium hydrogen phosphate, 0.24 gm potassium dihydrogen phosphate, 0.2 gm potassium chloride, 8.0g sodium chloride, pH 7.4 in 1 liter distilled water], containing 2% gelatin).

Groups of eight 6-week old Balb/c mice were inoculated intraperitoneally with 100 µl of recombinant minicells or recombinant killed *S*. *typhimurium* according to the schedule in Table 4. One group of 8 mice remained unvaccinated as negative controls. Mice were bled by intraocular bleeding before inoculation and at day 14 post-primary vaccination. On day 23, all animals were anaesthetized with an intraperitoneal injection of 12 mg sodium phenobarbitone and 1 ml of blood was collected by cardiac puncture before the mice were sacrificed. Blood was centrifuged at 3000 rpm for 10 minutes in a microfuge (Eppendorf, Hamburg, Germany) and serum was collected for ELISA assays. Throughout the experiment, animals were weighed weekly and observed daily for signs of toxicity.

**Table 4. Treatment allocation for in vivo gene delivery in Balb/c mice**

| **Group number** | **Animal number** | **Treatment** | **Dose (100 ul IP injection)** | **Dosing days** | **Bleeding days** |
|---|---|---|---|---|---|
| 1 | 1-8 | None- control | - | - | 1, 14, 23 |
| 2 | 9-16 | Recombinant minicells | 10⁸ minicells per dose | 1, 5 | 14,23 |
| 3 | 17-24 | Recombinant killed *S*. *typhimurium* | 10⁸ bacteria per dose | 1, 5 | 14,23 |
| 4 | 25-32 | Recombinant killed *S*. *typhimurium* | 10⁸ bacteria per dose | 1,5,8 | 14,23 |
| 5 | 33-40 | Recombinant killed *S*. *typhimurium* | 10⁹ bacteria per dose | 1, 5 | 14, 23 |
| 6 | 41-48 | Recombinant killed *S*. *typhimurium* | 10⁹ bacteria per dose | 1,5,8 | 14,23 |

ELISA assays were performed to determine whether antibody had been generated against GFP, that is, whether recombinant minicells were able to deliver the mammalian expression plasmid pEGFP-C1 *in-vivo. S*. *typhimurium* lipopolysaccharide (LPS) antibody levels were also determined for all groups. The indirect ELISA method was carried out as follows.

Ninety six (96)-well microtitre plates (Greiner GMBH, NYrtingen, Germany) were coated with 50 µl per well of 0.5 µg/ml rEGFP (Clontech, Palo Alto, CA, USA) or LPS antigen (Sigma). Plates were coated with 1% BSA (Sigma) as negative control. Plates were sealed and incubated overnight at 4°C. Plates were inverted to remove antigen solution, and 200 µl blocking buffer (0.05% Tween-20 [Sigma], 1% BSA in PBS) was added to each well before incubating for 2 hours at room temperature. Blocking buffer was removed and the plates were washed twice for 5 minutes with wash buffer (0.05% Tween-20, PBS). Serum samples were diluted 1 in 80 and 1 in 300 for EGFP and LPS respectively in blocking buffer. Next, 100 µl of sample was added to each well, and incubated 1 hour at room temperature with shaking. Plates were then washed with wash buffer 3 times for 5 minutes. Secondary antibodies, namely, alkaline phosphatase conjugated anti-mouse immunoglobulin (y- & light chains; Chemicon, Temicula, CA, USA), or AP-conjugated anti-LPS monoclonal antibody (IgG1 isotype, Biodesign International, Saco, Maine, USA) were diluted in blocking buffer, and 100 µl was added per well, followed by a 1 hour incubation at room temperature with shaking. Wells were washed three times with wash buffer, and 100 µl PNPP (*p*-nitrophenyl phosphate substrate; Zymed, San Francisco, CA, USA) was added. Absorbance at 405 nm was read after a 30 minute incubation at room temperature. The reaction was terminated by addition of 30 µl of 0.5 M NaOH. ELISA data significance was determined by Student t test (*p*).

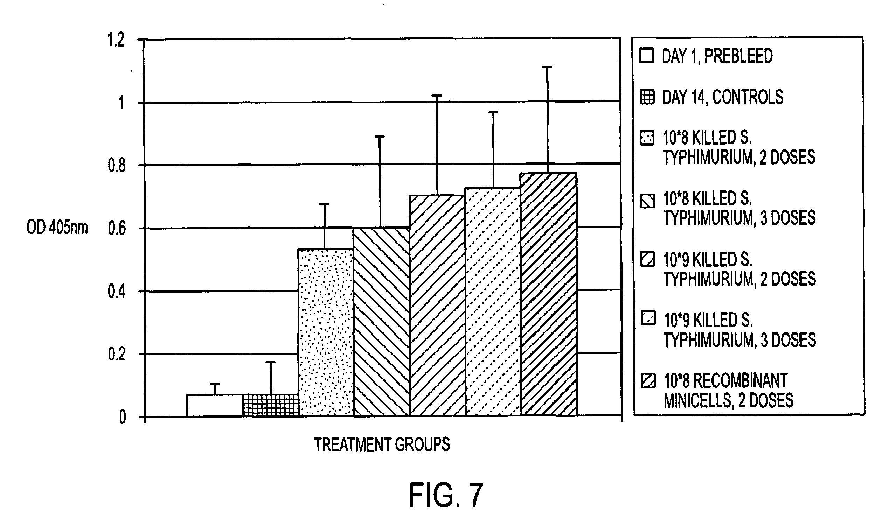

Results are shown in Figure 7. At 14 days post-vaccination, a strong and significant antibody response (*p* < 0.02 when compared to controls) to EGFP was observed in mice given 10⁸ recombinant minicells intraperitoneally, and the antibody response observed was greater than that obtained with the highest dose of killed *S*. *typhimurium.* Antibodies to EGFP protein would only be observed if the recombinant minicells bearing the mammalian expression vector pEGFP-C1 not only are engulfed by peritoneal macrophages but also are degraded in intracellular vacuoles (presumably, phagolysosomes), and that at least some plasmid DNA copies escaped the phagolysosomes and entered the mammalian cell nucleus. From the nucleus, EGFP mRNA would be produced and EGFP expressed in the cytoplasm. The EGFP would be a foreign protein in the macrophage and, hence, would be expected to be processed and peptides would be presented via MHC. This process would result in an antibody response to the EGFP peptides. Compared with the control killed *S*. *typhimurium,* the anti-EGFP antibody response was higher with the recombinant minicells.

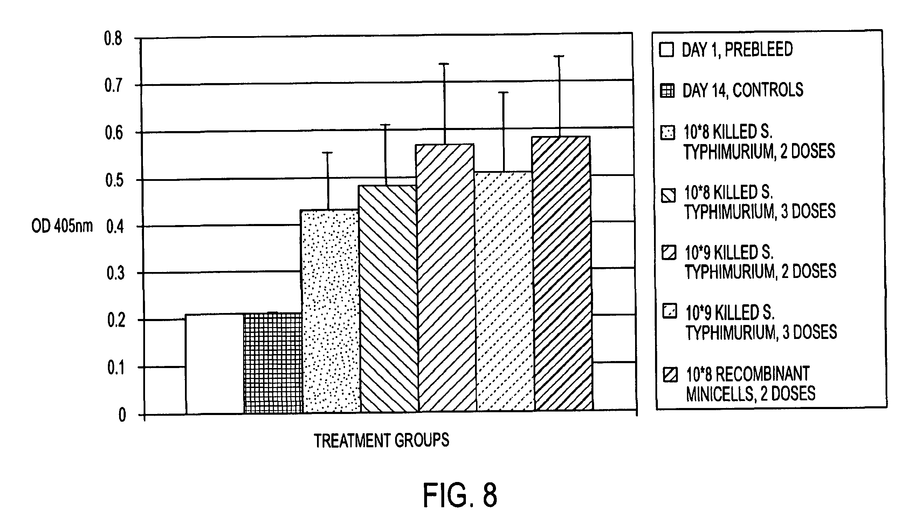

The anti-LPS response also was measured to determine the immune response to the gene therapy delivery vector, the recombinant minicells. The results showed that the anti-LPS antibody response was significant and similar for recombinant minicells (*p* = 0.0004) and killed *S*. *typhimurium* (*p* = 0.001). See Figure 8. This result indicated that the minicells had retained at least the LPS structure found on the parent bacterial cell surface. By day 23, the anti-EGFP antibody response was not different from the nonimmunized controls (for both recombinant minicells and killed *S*. *typhimurium*). This was not surprising because no booster immunizations had been administered to sustain the antibody response. The anti-LPS response at day 23 was similar to that seen at day 14. This is not unexpected because LPS is known to be a potent immunogen that induces high and sustained antibody titers.

### Example 10. Minicell-mediated gene delivery and gene expression in vivo in Balb/c mice with different dosing regimes

Recombinant minicells were prepared as in Example 3. Groups of eight, 6-week old Balb/c mice were inoculated intraperitoneally with 100 µl of recombinant minicells (containing plasmid pEGFP-C1; Table 2) according to the schedule shown in Table 5. One group of eight mice remained unvaccinated, as negative controls. Mice were bled by intraocular bleeding before inoculation and at day 14 post-primary vaccination and serum collected as in Example 9.

**Table 5. Treatment allocation for in vivo gene delivery with different dose regimes of recombinant minicells in Balb/c mice**

| **Group number** | **Animal number** | **Treatment** | **Dose (100µl IP injection)** | **Dosing days** | **Bleeding days** |
|---|---|---|---|---|---|
| 1 | 1-8 | None- control | - | - | 1, 14 |
| 2 | 9-16 | Recombinant minicells | 10⁸ minicells per dose | 1,4 | 14 |
| 3 | 17-24 | Recombinant minicells | 10⁸ minicells per dose | 1,4,8 | 14 |
| 4 | 25-32 | Recombinant minicells | 10⁹ minicells per dose | 1,4 | 14 |
| 5 | 33-40 | Recombinant minicells | 10⁹ minicells per dose | 1, 4, 8 | 14 |

ELISA assays were performed as previously described to determine whether antibody had been generated against GFP, and whether higher doses of recombinant minicells or three rather than two doses enabled the animal to mount a larger antibody response. *S*. *typhimurium* lipopolysaccharide (LPS) antibody levels were also determined for all groups.

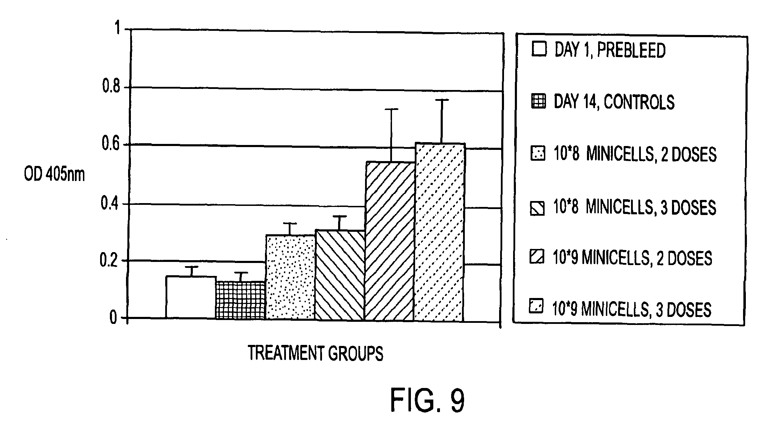

Results are shown in Figure 9. At 14 days post-vaccination, a very significant antibody response (*p* < 0.001 when compared to controls) to EGFP was observed in mice given 10⁸ recombinant minicells intraperitoneally. Mice inoculated with 10⁹ minicells showed an even greater antibody response to EGFP (*p* = 0.0006 compared to controls), and this dose gave significantly higher antibody levels than the lower dose of 10⁸ (*p* = 0.004). There was no significant difference in antibody response to EGFP protein when mice were given either two doses or three doses of recombinant minicells, suggesting that two doses may be enough to achieve gene therapy in this instance.

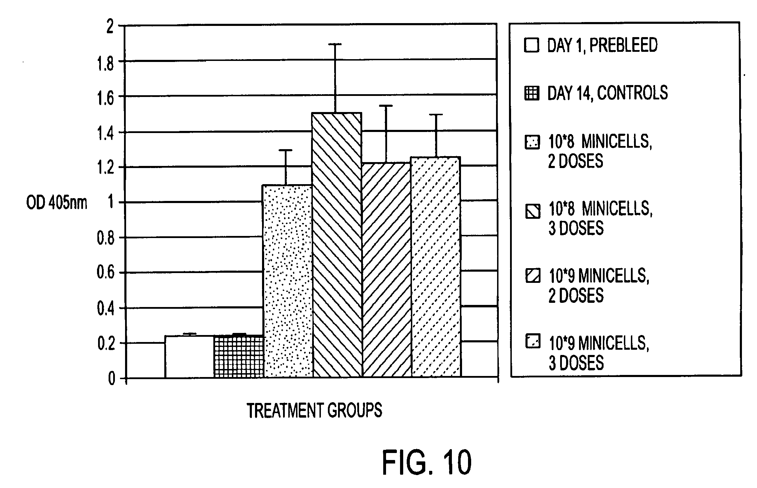

The anti-LPS response also was measured, to determine the immune response to the recombinant minicells. The results showed that the anti-LPS antibody response was significant (*p* = 0.0004). See Figure 10.

### CITED PUBLICATIONS

Balicki & Beutler, "Gene therapy of human disease," Medicine (Baltimore) 81: 69 (2002).

Brahmbhatt, "Cloning and molecular characterization of the rfb gene cluster of Salmonella typhimurium," Ph.D. Thesis, University of Adelaide, Australia (1987).

Britton et al., "Characterization of a prokaryotic SMC protein involved in chromosome partitioning," Genes Dev. 12: 1254 (1998).

Brückner, "A series of shuttle vectors for Bacillus subtilis and Escherichia coli," Gene 122: 187 (1992)

Catic et al., "Introduction of protein or DNA delivered via recombinant Salmonella typhimurium into the major histocompatibility complex class I presentation pathway of macrophages," Microbes Infect. 1: 133 (1999).

Ciliberto et al., "Cell-specific expression of a transfected human alpha 1-antitrypsin gene," Cell. 41: 531 (1985).

Clark-Curtiss & Curtiss, "Analysis of recombinant DNA using Escherichia coli minicells, " Methods Enzymol. 101: 347 (1983).

Courvalin et al., "Gene transfer from bacteria to mammalian cells," C.R. Acad. Sci. III 318: 1207 (1995).

Curiel et al., "Long-term inhibition of clinical and laboratory human immunodeficiency virus strains in human T-cell lines containing an HIV-regulated diphtheria toxin A chain gene," Hum. Gene Ther. 4: 741 (1993).

Davis et al., ADVANCED BACTERIAL GENETICS: A MANUAL FOR GENETIC ENGINEERING (Cold Spring Harbor Laboratory Press, 1980).

de Boer et al., "Roles of MinC and MinD in the site-specific septation block mediated by the MinCDE system of Escherichia coli," J. Bacteriol. 174: 63 (1992).

Dietrich et al., "Delivery of antigen-encoding plasmid DNA into the cytosol of macrophages by attenuated suicide Listeria monocytogenes, " Nature Biotechnol. 16: 181 (1998).

Dinges et al., "HIV-regulated diphtheria toxin A chain gene confers long-term protection against HIV type 1 infection in the human promonocytic cell line U937," Hum. Gene Ther. 6: 1437 (1995).

Dorward et al., "Export and intercellular transfer of DNA via membrane blebs of Neisseria gonorrhoeae," J. Bacteriol. 171: 2499 (1989).

Fire et al., "A modular set of lacZ fusion vectors for studying gene expression in Caenorhabditis elegans, " Gene 93: 189 (1990).

Firth et al., "Structure and function of the F factor and mechanism of conjugation," in ESCHERICHIA COLI AND SALMONELLA: CELLULAR AND MOLECULAR BIOLOGY 2nd ed. (1996), at pages 2377-2401.

Forbes, "Crossflow microfiltration," Australian J. Biotechnology 1: 30 (1987).

Frain et al., "Binding of a liver-specific factor to the human albumin gene promoter and enhancer," Mol. Cell Biol. 10: 991 (1990).

Frazer & Curtiss, "Production, properties and utility of bacterial minicells," Curr Top Microbiol. Immunol. 69: 1 (1975).

Freshner, ANIMAL CELL CULTURE: A PRACTICAL APPROACH 2nd ed. (Oxford/New York, IRL Press, Oxford University Press, 1992).

Gentschev et al., "Delivery of protein antigens and DNA by virulence-attenuated strains of Salmonella typhimurium and Listeria monocytogenes," J. Biotechnol. 83: 19 (2000).

Grillot-Courvalin et al., "Functional gene transfer from intracellular bacteria to mammalian cells," Nat. Biotechnol. 16: 862 (1998).

Grillot-Courvalin et al., "Wild-type intracellular bacteria deliver DNA into mammalian cells," Cell Microbiol. 4: 177 (2002).

Hanahan, "Studies on transformation of Escherichia coli with plasmids," J. Mol. Biol. 166: 557 (1983).

Haibin et al., "siRNA-mediated gene silencing in vitro and in vivo," Nat. Biotechnol. 20: 1006 (2002).

Hanahan, "Heritable formation of pancreatic beta-cell tumors in transgenic mice expressing recombinant insulin/simian virus 40 oncogenes," Nature 315: 115 (1985).

Harlow et al., "Cloning and characterization of the gsk gene encoding guanosine kinase of Escherichia coli, " J. Bacteriol. 177: 2236 (1995).

Harrison et al., "Inhibition of human immunodeficiency virus-1 production resulting from transduction with a retrovirus containing an HIV-regulated diphtheria toxin A chain gene," Hum. Gene Ther. 3: 461 (1992a).

Harrison et al., "Inhibition of HIV production in cells containing an integrated, HIV- regulated diphtheria toxin A chain gene," AIDS Res. Hum. Retroviruses 8: 39 (1992b).

Harry, "Bacterial cell division: Regulating Z-ring formation," Mol. Microbiol. 40: 795 (2001).

Hart, "Tissue specific promoters in targeting systematically delivered gene therapy," Semin. Oncol. 23: 154 (1996).

Hashimotoh-Gotoh et al., "Specific-purpose plasmid cloning vectors. I. Low copy number, temperature-sensitive, mobilization-defective pSC101-derived containment vectors," Gene 16: 227 (1981).

Heim et al., "Wavelength mutations and posttranslational autoxidation of green fluorescent protein," Proc. Nat'l. Acad. Sci. USA 91: 12501 (1994).

Hiraga et al., "Chromosome partitioning in Escherichia coli: novel mutants producing anucleate cells," J. Bacteriol. 171: 1496 (1989).

Hoiseth et al., "Aromatic-dependent Salmonella typhimurium are non-virulent and effective as live vaccines," Nature 291: 238 (1981).

Hu & Lutkenhaus, "Topological regulation of cell division in Escherichia coli involves rapid pole to pole oscillation of the division inhibitor MinC under the control of MinD and MinE," Mol. Microbiol. 34: 82 (1999).

Ireton et al., "spo0J is required for normal chromosome segregation as well as the initiation of sporulation in Bacillus subtilis," J. Bacteriol. 176: 5320 (1994).

Katabi et al., "Hexokinase Type II: A Novel Tumor Specific Promoter for Gene-Targeted Therapy Differentially Expressed and Regulated in Human Cancer Cells," Human Gene Therapy 10: 155 (1999).

Katsui et al., "Heat-induced blebbing and vesiculation of the outer membrane of Escherichia coli," J. Bacteriol. 151: 1523 (1982).

Kelsey et al., "Species- and tissue-specific expression of human alpha 1-antitrypsin in transgenic mice," Genes and Devel. 1: 161 (1987).

Kerem et al., "Identification of the cystic fibrosis gene: genetic analysis," Science 245: 1073 (1989).

Kihara et al., "Analysis of a FliM-FliN flagellar switch fusion mutant of Salmonella typhimurium," J. Bacteriol. 178: 4582 (1996).

Kurane et al., "Targeted Gene Transfer for Adenocarcinoma Using a Combination of Tumor specific Antibody and Tissue-specific Promoter," Jpn. J. Cancer Res. 89: 1212 (1998).

Leder et al., "Consequences of widespread deregulation of the c-myc gene in transgenic mice: multiple neoplasms and normal development," Cell 45: 485 (1986).

Levin et al., "Identification of Bacillus subtilis genes for septum placement and shape determination," J. Bacteriol. 174: 6717 (1992).

MacDonald et al., "Expression of the pancreatic elastase I gene in transgenic mice," Hepatology 7: 425 (1987).

Macrina et al., "A cloning vector able to replicate in Escherichia coli and Streptococcus sanguis," Gene 19: 345 (1982).

Marcil & Higgins. "Direct transfer of plasmid DNA from yeast to E. coli by electroporation," Nucl. Acids Res. 20: 917 (1992).

Mason et al., "The hypogonadal mouse: reproductive functions restored by gene therapy," Science 234: 1372 (1986).

Matsuzaki et al., "Interactions of an antimicrobial peptide, magainin 2, with outer and inner membranes of Gram-negative bacteria," Biochim Biophys. Acta. 1327: 119 (1997).

Miller & Mekalanos, "A novel suicide vector and its use in construction of insertion mutations: osmoregulation of outer membrane proteins and virulence determinants in Vibrio cholerae requires toxR," J. Bacteriol. 170: 2575 (1988).

Mori, "Complete and shotgun sequencing: yehV, minE, minD, minC, ypjA from Escherichia coli strain K12," direct submission to the DDBJ/EMBL/GenBank databases (1996).

Morton & Potter, "Rhabdomyosarcoma-specific expression of the herpes simplex virus thymidine kinase gene confers sensitivity to ganciclovir," J. Pharmacology & Exper. Therapeutics 286: 1066 (1998).

Okada et al., "Possible function of the cytoplasmic axial filaments in chromosomal segregation and cellular division of Escherichia coli," Sci. Prog. 77: 253 (1993-94).

Okada et al., "Cytoplasmic axial filaments in Escherichia coli cells: possible function in the mechanism of chromosome segregation and cell division," J. Bacteriol. 176: 917 (1994).

Pearson & Lipman, "Improved tools for biological sequence comparison," Proc. Nat'l. Acad. Sci. USA 85: 2444 (1988).

Pikaar et al., "Opsonic activities of surfactant proteins A and D in phagocytosis of gram-negative bacteria by alveolar macrophages," J. Infect. Dis. 172: 481 (1995).

Pinkert et al., "An albumin enhancer located 10 kb upstream functions along with its promoter to direct efficient, liver-specific expression in transgenic mice," Genes and Devel. 1: 268 (1987).

Prasher et al., "Using GFP to see the light," Trends in Genetics 11: 320 (1995).

Ragheb et al., "Inhibition of human immunodeficiency virus type 1 by Tat/Rev-regulated expression of cytosine deaminase, interferon alpha2, or diphtheria toxin compared with inhibition by transdominant Rev," Hum. Gene Ther. 10: 103 (1999).

Ralph & Nakoinz, "Antibody-dependent killing of erythrocyte and tumor targets by macrophage-related cell lines: Enhancement by PPD and LPS," J. Immunol. 119: 950 (1977).

Raskin & de Boer, "MinDE-dependent pole-to-pole oscillation of division inhibitor MinC in Escherichia coli, " J. Bacteriol. 181: 6419 (1999).

Readhead et al., "Myelin deficient mice: expression of myelin basic protein and generation of mice with varying levels of myelin," Cell 48: 703 (1987).

Reeve, "Use of minicells for bacteriophage-directed polypeptide synthesis," Methods Enzymol. 68: 493 (1979).

Reeve & Cornett, "Bacteriophage SPO1-induced macromolecular synthesis in minicells of Bacillus subtilis," J. Virol. 15: 1308 (1975).

Riezman, "Three clathrin-dependent budding steps and cell polarity," Trends in Cell Biology. 3: 330 (1993).

Riordan et al., "Identification of the cystic fibrosis gene: cloning and characterization of complementary DNA," Science 245: 1066 (1989).

Romano et al., "Gene transfer technology in therapy: current applications and future goals," Onocologist 3: 225 (1998).

Romano et al., "Gene transfer technology in therapy: current applications and future goals," Stem Cells. 17: 191 (1999).

Rommens et al., "Identification of the cystic fibrosis gene: Chromosome walking and jumping," Science 245: 1059 (1989).

Sambrook et al., MOLECULAR CLONING: LABORATORY MANUAL 2nd ed. (Cold Spring Harbor Laboratory Press, 1989).

Sancar et al., "Simple method for identification of plasmid-coded proteins," J. Bacteriol. 137: 692 (1979).

Sandvig & Deurs, "Endocytosis without clathrin," Trends in Cell Biology. 4: 275 (1994).

Seeliger, LISTERIOSIS, 2nd ed., at page 308 (Karger, 1961).

Shangara et al., "Suicide genes: past, present and future perspectives," Immunology Today 21: 48 (2000).

Shaw & Griffen, "Phagocytosis requires repeated triggering of macrophage phagocytic receptors during particle ingestion," Nature 289: 409 (1981).

Shigekawa & Dower, "Electroporation of eukaryotes and prokaryotes: A general approach to the introduction of macromolecules into cells," BioTechniques 6: 742 (1988).

Simon et al., "A broad host range mobilization system for in vivo genetic engineering: transposon mutagenesis in gram negative bacteria," Biotechnology 1: 784 (1983).

Sizemore et al., "Attenuated Shigella as a DNA delivery vehicle for DNA-mediated immunization," Science 270: 299 (1995).

Speert et al., "Functional characterization of macrophage receptors for In-vitro phagocytosis of unopsonized pseudomonas-aeruginosa," J. Clin. Invest. 82: 872 (1988).

Spencer, "Developments in suicide genes for preclinical and clinical applications," Molecular Therapeutics 2: 433 (2000).

Stewart & D'Ari, "Genetic and morphological characterization of an Escherichia coli chromosome segregation mutant," J. Bacteriol. 174: 4513 (1992).

Sullivan et al., "New shuttle vectors for Bacillus subtulis and Escherichia coli which allow rapid detection of inserted fragments," Gene 29: 21 (1984).

Swift et al., "Tissue-specific expression of the rat pancreatic elastase I gene in transgenic mice," Cell 38: 639 (1984).

Trieu-Cuot et al., "Shuttle vectors containing a multiple cloning site and a lacZ alpha gene for conjugal transfer of DNA from Escherichia coli to gram-positive bacteria," Gene 102: 99 (1991).

Wachi et al., "New mre genes mreC and mreD, responsible for formation of the rod shape of Escherichia coli cells," J. Bacteriol. 171: 6511 (1989).

Wadhwa et al., "Cancer gene therapy: Scientific basis," Ann. Rev. Med. 53: 437 (2002).

Wright & Jong, "Interferon-gama depresses binding of ligand by c3b and c3bi receptors on cultured human monocytes, an effect reversed by fibronectin," Experimental Medi. 163: 1245 (1986).

Yanisch-Perron et al., "Improved M13 phage cloning vectors and host strains: nucleotide sequences of the M13mp18 and pUC19 vectors," Gene 33: 103 (1985).

Yazawa et al., "Current progress in suicide gene therapy for cancer," World J. Surg. 26: 783 (2002).

## Claims

1. A composition comprising (i) recombinant, intact bacterial minicells and (ii) a pharmaceutically acceptable carrier therefor, wherein said intact bacterial minicells contain a plasmid comprising a DNA sequence encoding a therapeutic peptide or polypeptide expression product, and wherein said composition is free of contaminants 0.2 µm or less in size.

2. A composition according to claim 1, wherein said composition contains fewer than about 1 contaminating parent bacterial cell per 10⁷ minicells.

3. A composition according to claim 1, wherein said composition contains fewer than about 1 contaminating parent bacterial cell per 10⁸ minicells.

4. A composition according to claim 1, wherein said composition contains about 1 contaminating parent bacterial cell per 10⁹ minicells.

5. A composition consisting essentially of recombinant, intact bacterial minicells that contain a plasmid comprising a DNA sequence encoding a therapeutic peptide or polypeptide expression product, wherein said composition is free of contaminants 0.2 µm or less in size.

6. A genetic transformation method that comprises (i) providing recombinant, intact bacterial minicells that contain a plasmid comprising a DNA sequence encoding a therapeutic peptide or polypeptide expression product, wherein said bacterial minicells are free of contaminants 0.2 µm or less in size and (ii) bringing said preparation of intact bacterial minicells into contact *in vitro* with non-phagocytic endocytosis-competent mammalian cells, such that said bacterial minicells are engulfed by said mammalian cells, whereby said mammalian cells produce said expression product.

7. A purification method that comprises passing a sample containing intact bacterial minicells (i) over a series of cross-flow filters and then (ii) through a dead-end filter, whereby said intact bacterial minicells are separated from contaminants in said sample to obtain a purified minicell preparation.

8. A method according to claim 7, further comprising the step of treating said purified minicell preparation with an antibiotic.

9. A method according to claim 7 or 8, further comprising a preliminary step of performing differential centrifugation on said sample containing bacterial minicells.

10. A method according to any one of claims 7-9, wherein said series of cross-flow filters comprises at least one filter employing a pore size greater than or equal to about 0.45 µm, and at least one filter employing a pore size less than or equal to about 0.2 µm.

11. A method according to claim 10, wherein said dead-end filter employs a pore size of about 0.45 µm.

12. A method according to claim 11, wherein said series of cross-flow filters comprises at least two filters employing a pore size of about 0.45 µm, and at least one filter employing a pore size of about 0.2 µm.

13. A method according to claim 12, further comprising the step of treating said purified minicell preparation with an antibiotic.

14. Use of recombinant, intact bacterial minicells in the preparation of a medicament that comprises intact bacterial minicells containing a plasmid comprising a DNA sequence encoding a therapeutic peptide or polypeptide expression product, wherein said medicament is free of contaminants 0.2 µm or less in size , for use in a method of (i) treating a disease, allergy or infection or (ii) modifying a trait, by administration of said medicament to a cell, tissue, or organ, wherein said disease is selected from (1) cancer; (2) an acquired disease selected from AIDS, pneumonia and emphysema; and (3) a condition engendered by an inborn error of metabolism, the allergy or infection is treated by modification of an immune response, and said trait is fertility.

15. Recombinant, intact bacterial minicells containing a plasmid comprising a DNA sequence encoding a therapeutic peptide or polypeptide expression product, wherein said bacterial minicells are free of contaminants 0.2 µm or less in size , for use as a pharmaceutical.

16. A composition according to any one of claims 1-6, for use as a pharmaceutical.

17. A composition according to any one of claims 1-5 for use in a method of (i) treating a disease, allergy or infection or (ii) modifying a trait, by administration of said composition to a cell, tissue, or organ, wherein said disease is selected from (1) cancer; (2) an acquired disease selected from AIDS, pneumonia and emphysema; and (3) a condition engendered by an inborn error of metabolism, the allergy or infection is treated by modification of an immune response, and said trait is fertility.

## Patentansprüche

1. Zusammensetzung, umfassend (i) rekombinante, intakte bakterielle Minizellen und (ii) einen dafür pharmazeutisch verträglichen Träger, wobei die intakten bakteriellen Minizellen ein Plasmid enthalten, welcher eine DNA-Sequenz umfasst, die ein therapeutisches Peptid-oder Polypeptid-Expressionsprodukt kodiert, und wobei die Zusammensetzung frei von 0,2 µm oder weniger großen Kontaminanten ist.

2. Zusammensetzung nach Anspruch 1, wobei die Zusammensetzung weniger als ungefähr 1 kontaminierende bakterielle Mutterzelle pro 10⁷ Minizellen enthält.

3. Zusammensetzung nach Anspruch 1, wobei die Zusammensetzung weniger als ungefähr 1 kontaminierende bakterielle Mutterzelle pro 10⁸ Minizellen enthält.

4. Zusammensetzung nach Anspruch 1, wobei die Zusammensetzung ungefähr 1 kontaminierende bakterielle Mutterzelle pro 10⁹ Minizellen enthält.

5. Zusammensetzung, die im Wesentlichen aus rekombinanten, intakten bakteriellen Minizellen besteht, welche ein Plasmid enthalten, das eine DNA-Sequenz umfasst, die ein therapeutisches Peptid- oder Polypeptid-Expressionsprodukt kodiert, wobei die Zusammensetzung frei von 0,2 µm oder weniger großen Kontaminanten ist.

6. Genetisches Transformationsverfahren, welches umfasst (i) Bereitstellen von rekombinanten, intakten bakteriellen Minizellen, welche ein Plasmid enthalten, das eine DNA-Sequenz umfasst, die ein therapeutisches Peptid- oder Polypeptid-Expressionsprodukt kodiert, wobei die bakteriellen Minizellen frei von 0,2 µm oder weniger großen Kontaminanten sind und (ii) Inkontaktbringen der Präparation von intakten bakteriellen Minizellen *in vitro* mit nicht-phagozytischen Endozytose-kompetenten Säugerzellen, so dass die bakteriellen Minizellen von den Säugerzellen verschlungen werden, wodurch die Säugerzellen das Expressionsprodukt produzieren.

7. Reinigungsverfahren, welches das Weiterleiten einer intakte bakterielle Minizellen enthaltende Probe (i) über eine Reihe von Cross-Flow-Filtern und dann (ii) durch einen Dead-End-Filter umfasst, wodurch die intakten bakteriellen Minizellen von den Kontaminanten in der Probe getrennt werden, um eine gereinigte Minizell-Präparation zu erhalten.

8. Verfahren nach Anspruch 7, ferner umfassend den Schritt zur Behandlung der gereinigten Minizell-Präparation mit einem Antibiotikum.

9. Verfahren nach Anspruch 7 oder 8, ferner umfassend einen einleitenden Schritt zur Durchführung von differentieller Zentrifugation der Probe, die bakterielle Minizellen enthält.

10. Verfahren nach irgendeinem der Ansprüche 7 - 9, wobei die Reihe von Cross-Flow-Filtern mindestens einen Filter umfasst, welcher eine Porengröße größer als oder gleich ungefähr 0,45 µm verwendet, und mindestens einen Filter, welcher eine Porengröße kleiner als oder gleich ungefähr 0,2 µm verwendet.

11. Verfahren nach Anspruch 10, wobei der Dead-End-Filter eine Porengröße von ungefähr 0,45 µm verwendet.

12. Verfahren nach Anspruch 11, wobei die Reihe von Cross-Flow-Filtern mindestens zwei Filter umfasst, welche eine Porengröße von ungefähr 0,45 µm verwenden, und mindestens einen Filter, der eine Porengröße von ungefähr 0,2 µm verwendet.

13. Verfahren nach Anspruch 12, ferner umfassend den Schritt zur Behandlung der gereinigten Minizell-Präparation mit einem Antibiotikum.

14. Verwendung von rekombinanten, intakten bakteriellen Minizellen in der Herstellung von einem Medikament, welches intakte bakterielle Minizellen umfasst, die ein Plasmid enthalten, das eine DNA-Sequenz umfasst, die ein therapeutisches Peptid- oder Polypeptid-Expressionsprodukt kodiert, wobei das Medikament frei von 0,2 µm oder weniger großen Kontaminanten ist, zur Verwendung in einem Verfahren zur (i) Behandlung einer Erkrankung, Allergie oder Infektion oder (ii) Änderung eines Merkmals durch Verabreichen des Medikaments an eine Zelle, Gewebe, oder Organ,
wobei die Erkrankung ausgewählt ist aus (1) Krebs; (2) einer erworbenen Erkrankung ausgewählt aus AIDS, Lungenentzündung und Emphysem; und (3) einem durch eine angeborene Stoffwechselstörung hervorgerufene Zustand,
die Allergie oder Infektion durch Änderung einer Immunantwort behandelt wird,
und das Merkmal Fertilität ist.

15. Rekombinante, intakte bakterielle Minizellen, welche ein Plasmid enthalten, das eine DNA-Sequenz umfasst, welche ein therapeutisches Peptid- oder Polypeptid-Expressionsprodukt kodiert, wobei die bakteriellen Minizellen frei von 0,2 µm oder weniger großen Kontaminanten sind, zur Verwendung als ein Arzneimittel.

16. Zusammensetzung nach irgendeinem der Ansprüche 1 - 6, zur Verwendung als ein Arzneimittel.

17. Zusammensetzung nach irgendeinem der Ansprüche 1 - 5 zur Verwendung in einem Verfahren zur (i) Behandlung einer Erkrankung, Allergie oder Infektion oder (ii) Änderung eines Merkmals durch Verabreichen der Zusammensetzung an eine Zelle, Gewebe, oder Organ,
wobei die Erkrankung ausgewählt ist aus (1) Krebs; (2) einer erworbenen Erkrankung ausgewählt aus AIDS, Lungenentzündung und Emphysem; und (3) einem durch eine angeborene Stoffwechselstörung hervorgerufene Zustand,
die Allergie oder Infektion durch Änderung einer Immunantwort behandelt wird,
und das Merkmal Fertilität ist.

## Revendications

1. Une composition comprenant (i) des mini-cellules bactériennes recombinantes intactes et (ii) un support pharmaceutiquement acceptable, dans lequel les mini-cellules bactériennes intactes contiennent un plasmide comprenant une séquence d'ADN encodant pour un produit d'expression thérapeutique peptide ou polypeptide, et dans laquelle cette composition est libre de contaminants ayant une taille de 0,2 µm ou moins.

2. Une composition selon la revendication 1, dans laquelle la composition contient moins d'environ 1 cellule bactérienne parente contaminante pour 10⁷ mini-cellules.

3. Une composition selon la revendication 1, dans laquelle la composition contient moins d'environ 1 cellule bactérienne parente contaminante pour 10⁸ mini-cellules.

4. Une composition selon la revendication 1, dans laquelle la composition contient environ 1 cellule bactérienne parente contaminante pour 10⁹.

5. Composition consistant essentiellement en mini-cellules bactériennes recombinantes intactes qui contient un plasmide comprenant une séquence d'ADN encodant pour un produit d'expression thérapeutique peptide ou polypeptide, dans laquelle la composition est libre de contaminants ayant une taille de 0,2 µm ou moins.

6. Méthode de transformation génétique qui comprend (i) la fourniture de mini-cellules bactériennes recombinantes intactes qui contiennent un plasmide comprenant une séquence d'ADN encodant pour un produit d'expression thérapeutique peptide ou polypeptide, dans laquelle les mini-cellules bactériennes sont libres de contaminants ayant une taille de 0,2 µm ou moins, et (ii) la mise en contact in vitro de la préparation des mini-cellules bactériennes intactes avec des cellules mammaliennes endocytose-compétentes non-phagocytiques et en telle sorte que les mini-cellules bactériennes sont englouties pars les cellules mammifères, dans laquelle les cellules mammaliennes produisent le produit d'expression.

7. Méthode de purification qui comprend le passage d'un échantillon comprenant des mini-cellules bactériennes intactes (i) sur une série de filtre à flot transversal et (ii) à travers un filtre cul-de-sac où les mini-cellules bactériennes intactes sont séparées des contaminants dans l'échantillon, afin d'obtenir une préparation de mini-cellules purifiées.

8. Méthode selon la revendication 7, qui comprend encore l'étape consistant à traiter la préparation des mini-cellules purifiées avec un antibiotique.

9. Méthode selon les revendications 7 ou 8, qui comprend en outre une étape préliminaire consistant à soumettre l'échantillon contenant les mini-cellules bactériennes à une centrifugation différentielle.

10. Méthode selon l'une des revendications 7 à 9, dans laquelle les séries de filtres transversaux comprennent au moins un filtre utilisant une taille de pore supérieure à ou égal à environ 0.45 µm, et au moins un filtre utilisant une taille de pore inférieure à ou égal à environ 0,2 µm.

11. Méthode selon la revendication 10, dans laquelle ce filtre cul-de-sac utilise une taille de pore d'environ 0.45 µm.

12. Méthode selon la revendication 11, dans laquelle les séries de filtres transversaux comprennent au moins deux filtres utilisant une taille de pore d'environ 0.45 µm, et au moins un filtre utilisant une taille de pore d'environ 0,2 µm.

13. Méthode selon la revendication 12, comprenant en outre l'étape consistant à traiter la préparation de mini-cellules purifiées avec un antibiotique.

14. Utilisation de mini-cellules bactériennes recombinantes intactes dans la préparation d'un médicament qui comprend des mini-cellules bactériennes intactes comprenant un plasmide comprenant une séquence d'ADN encodant pour un produit d'expression thérapeutique peptide ou polypeptide, dans laquelle le médicament est libre de contaminants ayant une taille de 0,2 µm ou moins, à utiliser dans une méthode (i) pour traiter une maladie, une allergie ou une infection, ou (ii) pour modifier un trait par administration de ce médicament à une cellule, un tissu ou un organe, la maladie éàtant choisie dans le groupe comprenant (1) le cancer; (2) une maladie acquise choisie parmi le SIDA, la pneumonie et l'emphysème ; et (3) une condition générée par une erreur innée de métabolisme, l'allergie ou l'infection est traitée par modification d'une réponse immune, et ce trait est la fertilité.

15. Mini-cellules bactériennes recombinantes intactes contenant un plasmide comprenant une séquence d'ADN encodant pour un produit d'expression thérapeutique peptide ou polypeptide, dans laquelle les mini-cellules bactériennes sont libres de contaminant ayant taille de moins de 0.2 µm, à utiliser comme produit pharmaceutique.

16. Composition selon l'une des revendications 1 à 6, à utiliser comme produit pharmaceutique.

17. Composition selon l'une des revendications 1 à 5, pour utilisation dans une méthode de (i) traitement d'une maladie, d'une allergie ou d'une infection ou (ii) pour modifier un trait, par administration de la composition à une cellule, un tissu ou un organe, la maladie étant choisie dans le groupe comprenant (1) le cancer; (2) une maladie acquise choisie parmi le SIDA, la pneumonie et l'emphysème; et (3) une condition générée par une erreur innée de métabolisme, l'allergie ou l'infection est traitée par modification d'une réponse immune, et ce trait est la fertilité.
